(19) Europäisches Patentamt
European Patent Office
Office européen des brevets

(11) EP 4 442 677 A1

(12) EUROPEAN PATENT APPLICATION
published in accordance with Art. 153(4) EPC

(43) Date of publication:
09.10.2024 Bulletin 2024/41

(21) Application number: 22901299.2

(22) Date of filing: 29.11.2022

(51) International Patent Classification (IPC):
*C07C 69/54* (2006.01) *C07C 39/06* (2006.01)
*C07D 241/12* (2006.01) *C08F 2/44* (2006.01)
*C08F 20/14* (2006.01)

(52) Cooperative Patent Classification (CPC):
(C-Sets available)
**C08K 5/3462; C07C 67/62; C07D 241/12; C08F 2/44; C08F 20/14; C08K 5/10** (Cont.)

(86) International application number:
PCT/JP2022/043975

(87) International publication number:
WO 2023/100867 (08.06.2023 Gazette 2023/23)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 30.11.2021 JP 2021194503

(71) Applicant: Mitsubishi Chemical Corporation
Tokyo 100-8251 (JP)

(72) Inventors:
- KURIHARA, Yu
Tokyo 100-8251 (JP)
- SUZUKI, Tatsuya
Tokyo 100-8251 (JP)
- KATO, Yuki
Tokyo 100-8251 (JP)
- NINOMIYA, Wataru
Tokyo 100-8251 (JP)
- KAKIMOTO, Maiko
Tokyo 100-8251 (JP)

(74) Representative: Hoffmann Eitle
Patent- und Rechtsanwälte PartmbB
Arabellastraße 30
81925 München (DE)

# (54) METHYL METHACRYLATE - CONTAINING COMPOSITION, STORAGE METHOD FOR METHYL METHACRYLATE-CONTAINING COMPOSITION, AND PRODUCTION METHOD FOR METHYL METHACRYLATE POLYMER

(57) The purpose of the present invention is to provide a methyl methacrylate-containing composition with high quality stability during storage. This can be solved with a methyl methacrylate-containing composition, a pyrazine compound represented by Formula (1) (component A1), and a polymerization inhibitor (Component B1), in which the concentration of methyl methacrylate is from 99 to 99.99% by mass.

EP 4 442 677 A1

(52) Cooperative Patent Classification (CPC): (Cont.)

C-Sets
**C07C 67/62, C07C 69/54**

**Description**

TECHNICAL FIELD

The present invention relates to a methyl methacrylate-containing composition, a method of storing a methyl methacrylate-containing composition, and a method of producing methyl methacrylate polymer.

BACKGROUND ART

Methyl methacrylate (hereinafter also referred to as "MMA") is known to be an extremely useful substance used as a raw material of various applications and types of polymers. For example, polymethyl methacrylate, which is a homopolymer of methyl methacrylate, is used in signboards, lighting equipment, automotive parts, construction-related materials, light guiding panels for flat displays, light diffusion plates, and the like, taking advantage of its excellent transparency, weather resistance, and other properties. In addition, copolymers of methyl methacrylate and other monomers are used in paints, adhesives, resin modifiers, artificial marble, latices for paper, and the like. Various methods have been developed for industrially producing methyl methacrylate, and for example, the acetone cyanohydrin (ACH) method, the new acetone cyanohydrin (new ACH) method, the C4 direct oxidation method, the direct methyl esterification method, the ethylene method, and the new ethylene method are known (Non-Patent Document 1). In these production methods, methyl methacrylate of a quality suitable for the intended use is obtained by performing purification such as distillation to remove unreacted raw materials and by-products contained in the generated methyl methacrylate.

Since methyl methacrylate has a tendency to polymerize, it is known that the quality of methyl methacrylate is maintained by adding a polymerization inhibitor when producing methyl methacrylate or storing produced methyl methacrylate (Non-Patent Document 2). For example, Patent Document 1 describes that methyl ether of hydroquinone (MEHQ) is particularly preferable among various polymerization inhibitors. Patent Document 2 describes that N,N'-dialkyl-p-phenylenediamine and N-oxyl are preferable among various polymerization inhibitors. Patent Document 3 describes distillation of methyl methacrylate in the presence of a phenol polymerization inhibitor. Patent Document 4 describes use of a diphenylamine derivative as a polymerization inhibitor. Patent Document 5 describes use of a benzene triamine derivative as a polymerization inhibitor.

Patent Document 6 describes a method using a composition containing methyl methacrylate and a vinyl compound when producing a copolymer comprising methyl methacrylate.

PRIOR ART DOCUMENTS

PATENT DOCUMENTS

Patent Document 1: JP 2004-155757 A
Patent Document 2: JP 2005-502695 A
Patent Document 3: JP H10-504553 A
Patent Document 4: JP 2002-533309 A
Patent Document 5: JP 2002-513034 A
Patent Document 6: JP S50-37882 A

NON-PATENT DOCUMENTS

Non-Patent Document 1: Toru Kuroda, "Development of Catalyst for Producing Methyl Methacrylate", Catalysts & Catalysis, 45 (5), 366-371, 2003, Catalysis Society of Japan

Non-Patent Document 2: Takayuki Otsu, "On the Functions of Polymerization Inhibitors", Synthetic Organic Chemistry, 33 (8), 634-640, 1975, The Society of Synthetic Organic Chemistry, Japan

SUMMARY OF THE INVENTION

PROBLEMS TO BE SOLVED BY THE INVENTION

However, even in cases where polymerization inhibitors described in Patent Documents 1 to 5 are added, the quality of methyl methacrylate may deteriorate during storage. Patent Document 6 is a document on the production of copolymers and does not describe the storage stability of methyl methacrylate. In view of the above circumstances, the

purpose of the present invention is to provide a methyl methacrylate-containing composition with high quality stability during storage.

MEANS FOR SOLVING THE PROBLEMS

In order to achieve the above purposes, the present inventors have intensively studied. As a result, they found that, in methyl methacrylate with the quality deteriorated during storage, the concentration of methyl methacrylate is reduced, and methyl methacrylate dimers and methyl pyruvate are generated. The presence of methyl methacrylate dimers in methyl methacrylate may change the structure of methyl methacrylate polymer obtained by polymerization to adversely affect its physical properties. Furthermore, the presence of methyl pyruvate in methyl methacrylate causes coloration of methyl methacrylate polymer obtained by polymerization. The present inventors have found that inclusion of a pyrazine compound represented by a specific structural formula in a methyl methacrylate-containing composition results in improved quality stability during storage, and reduced the generation of methyl methacrylate dimers and methyl pyruvate, thereby completing the present invention.

Accordingly, the present invention provides the following [1] to [39]:

[1]: A methyl methacrylate-containing composition, comprising:

methyl methacrylate;
a pyrazine compound represented by the following Formula (1) (component A1); and
a polymerization inhibitor (component B1),
wherein the concentration of methyl methacrylate is from 99 to 99.99% by mass,

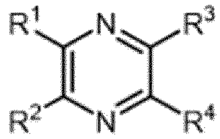

(1)

wherein, in Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group;

[2]: The methyl methacrylate-containing composition according to [1], wherein when the concentration of the component A1 is MA1 (umol/L), and the concentration of the component B1 is MB1 (umol/L), the MB1/MA1 is from 0.001 to 100;

[3]: The methyl methacrylate-containing composition according to [1] or [2], wherein when the concentration of the component A1 is MA1 (umol/L), the MA1 is from 1 to 65,000 μmol/L;

[4]: The methyl methacrylate-containing composition according to [3], wherein the MA1 is from 10 to 30,000 μmol/L;

[5]: The methyl methacrylate-containing composition according to any of [1] to [4], wherein when the concentration of the component B1 is MB1 (umol/L), the MB1 is from 1 to 7,000 μmol/L;

[6]: The methyl methacrylate-containing composition according to [5], wherein the MB1 is from 10 to 1,000 μmol/L;

[7]: The methyl methacrylate-containing composition according to any of [1] to [6], wherein the molecular weight of the component A1 is 1,000 or less;

[8]: The methyl methacrylate-containing composition according to any of [1] to [7], wherein the component B1 is at least one polymerization inhibitor selected from the group consisting of a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound;

[9]: The methyl methacrylate-containing composition according to any of [1] to [8], wherein the component B1 is at least one polymerization inhibitor selected from the group consisting of a phenol compound, an N-oxyl compound, an amine compound, a phosphorus-containing compound, and a sulfur-containing compound;

[10]: The methyl methacrylate-containing composition according to any of [1] to [9], wherein the component B1 is at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine;

[11]: The methyl methacrylate-containing composition according to any of [1] to [10], wherein in the Formula (1), $R^1$, $R^2$, $R^3$, and $R^4$ are each independently a hydrogen atom, a $C_{1-5}$ alkyl group, a $C_{2-5}$ alkenyl group, a $C_{1-12}$ aryl

group, a $C_{1-6}$ alkoxy group, a $C_{0-6}$ amino group, a $C_{1-6}$ monovalent group containing a carbonyl group, a $C_{1-5}$ alkylthio group, or a $C_{6-10}$ arylthio group;

[12]: The methyl methacrylate-containing composition according to any of [1] to [11], wherein in the Formula (1), $R^1$, $R^2$, $R^3$, and $R^4$ are each independently a hydrogen atom, a $C_{1-5}$ alkyl group, or a $C_{1-6}$ alkoxy group;

[13]: The methyl methacrylate-containing composition according to any of [1] to [12], wherein in the Formula (1), $R^1$, $R^2$, $R^3$, and $R^4$ are each independently a hydrogen atom, a methyl group, an ethyl group, an n-propyl group, an isopropyl group, or a methoxy group;

[14]: The methyl methacrylate-containing composition according to any of [1] to [13], wherein the concentration of methyl methacrylate is from 99.8 to 99.99% by mass;

[15]: The methyl methacrylate-containing composition according to any of [1] to [14], wherein the composition does not contain diacetyl, or the concentration of diacetyl contained is 55 μmol/L or less;

[16]: A methyl methacrylate-containing composition, comprising:

methyl methacrylate; and
a pyrazine compound represented by the following Formula (2) (component A2),
wherein the concentration of methyl methacrylate is from 99 to 99.99% by mass,

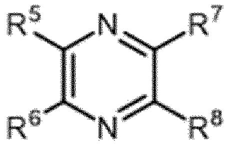

(2)

wherein, in Formula (2) above, $R^5$, $R^6$, $R^7$, and $R^8$ each independently represent a hydrogen atom, an alkyl group, an alkenyl group having a carbon number of 3 or more, an aryl group, a hydroxy group, an alkoxy group, an amino group, a monovalent group containing a carbonyl group, an alkylthio group, or an arylthio group;

[17]: The methyl methacrylate-containing composition according to [16], wherein when the concentration of the component A2 is MA2 (umol/L), the MA2 is from 1 to 65,000 μmol/L;

[18]: The methyl methacrylate-containing composition according to [17], wherein the MA2 is from 10 to 30,000 μmol/L;

[19]: The methyl methacrylate-containing composition according to any of [16] to [18], wherein the molecular weight of the component A2 is 1,000 or less;

[20]: The methyl methacrylate-containing composition according to any of [16] to [19], wherein in the Formula (2), $R^5$, $R^6$, $R^7$, and $R^8$ are each independently a hydrogen atom, a $C_{1-5}$ alkyl group, a $C_{3-5}$ alkenyl group, a $C_{1-12}$ aryl group, a $C_{1-6}$ alkoxy group, a $C_{0-6}$ amino group, a $C_{1-6}$ monovalent group containing a carbonyl group, a $C_{1-5}$ alkylthio group, or a $C_{6-10}$ arylthio group;

[21]: The methyl methacrylate-containing composition according to any of [16] to [20], wherein in the Formula (2), $R^5$, $R^6$, $R^7$, and $R^8$ are each independently a hydrogen atom, a $C_{1-5}$ alkyl group, or a $C_{1-6}$ alkoxy group;

[22]: The methyl methacrylate-containing composition according to any of [16] to [21], wherein in the Formula (2), $R^5$, $R^6$, $R^7$, and $R^8$ are each independently a hydrogen atom, a methyl group, an ethyl group, an n-propyl group, an isopropyl group, or a methoxy group;

[23]: The methyl methacrylate-containing composition according to any of [16] to [22], wherein the concentration of methyl methacrylate is from 99.8 to 99.99% by mass;

[24]: The methyl methacrylate-containing composition according to any of [16] to [23], wherein the composition does not contain diacetyl, or the concentration of diacetyl contained is 55 μmol/L or less;

[25]: A methyl methacrylate-containing composition, comprising:

methyl methacrylate;
a pyrazine compound represented by the following Formula (1) (component A1); and
an ester compound having an α-hydrogen represented by the following Formula (3) (component B2),
wherein the concentration of methyl methacrylate is from 99 to 99.99% by mass,

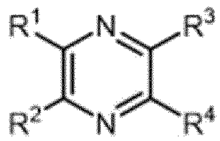

(1)

wherein, in Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group;

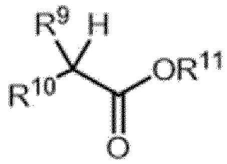 (3)

wherein in Formula (3) above, $R^9$ and $R^{10}$ each independently represent a hydrogen atom, an alkyl group, an alkenyl group, a hydroxy group, an alkoxy group, an amino group, a monovalent group containing a carbonyl group, or an alkylthio group,
$R^{11}$ represents an alkyl group or an aryl group, and
$R^9$ and $R^{10}$, $R^{10}$ and $R^{11}$, and $R^{11}$ and $R^9$ each may be linked to each other to form a ring;

[26]: The methyl methacrylate-containing composition according to [25], wherein when the concentration of the component A1 is MA1 (umol/L), and the concentration of the component B2 is MB2 (umol/L), the MB2/MA1 is from 0.01 to 1,000;
[27]: The methyl methacrylate-containing composition according to [25] or [26], wherein when the concentration of the component A1 is MA1 (umol/L), the MA1 is from 1 to 65,000 μmol/L;
[28]: The methyl methacrylate-containing composition according to any of [25] to [27], wherein when the concentration of the component B2 is MB2 (umol/L), the MB2 is from 1 to 50,000 μmol/L;
[29]: The methyl methacrylate-containing composition according to any of [25] to [28], wherein in the Formula (3), $R^9$ and $R^{10}$ are each independently a hydrogen atom, a $C_{1-5}$ alkyl group, a hydroxy group, a $C_{1-6}$ alkoxy group, a $C_{0-6}$ amino group, a $C_{1-6}$ monovalent group containing a carbonyl group, or a $C_{1-5}$ alkylthio group, and $R^{11}$ is a $C_{1-5}$ alkyl group or a $C_{1-12}$ aryl group;
[30]: The methyl methacrylate-containing composition according to any of [25] to [29], wherein the concentration of methyl methacrylate is from 99.8 to 99.99% by mass;
[31]: A methyl methacrylate-containing composition, comprising:

methyl methacrylate;
a pyrazine compound represented by the following Formula (1) (component A1); and
an α,β-unsaturated carbonyl compound represented by the following Formula (4) (component B3),
wherein the concentration of methyl methacrylate is from 99 to 99.99% by mass,

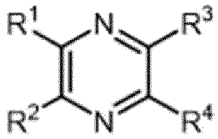 (1)

wherein, in Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group;

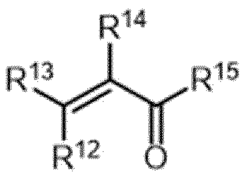 (4)

wherein, in Formula (4) above, $R^{12}$, $R^{13}$, and $R^{14}$ each independently represent a hydrogen atom, an alkyl group, an alkenyl group, an aryl group, a hydroxy group, an alkoxy group, an amino group, a monovalent group containing a carbonyl group, an alkylthio group, or an arylthio group,
$R^{15}$ represents an alkyl group, an alkenyl group, an aryl group, a hydroxy group, an alkoxy group, an amino

group, a monovalent group containing a carbonyl group, an alkylthio group, or an arylthio group,
$R^{12}$ and $R^{13}$, $R^{13}$ and $R^{14}$, and $R^{14}$ and $R^{15}$ each may be linked to each other to form a ring,
except when $R^{12}$ = H, $R^{13}$ = H, $R^{14}$ = $CH_3$, and $R^{15}$ = $OCH_3$, that is, when the α,β-unsaturated carbonyl compound represented by Formula (4) is methyl methacrylate, and
H represents a hydrogen atom, C represents a carbon atom, and O represents an oxygen atom;

[32]: The methyl methacrylate-containing composition according to [31], wherein when the concentration of the component A1 is MA1 (umol/L), and the concentration of the component B3 is MB3 (umol/L), the MB3/MA1 is from 0.01 to 1,000;
[33]: The methyl methacrylate-containing composition according to [31] or [32], wherein when the concentration of the component A1 is MA1 (umol/L), the MA1 is from 1 to 65,000 μmol/L;
[34]: The methyl methacrylate-containing composition according to any of [31] to [33], wherein when the concentration of the component B3 is MB3 (umol/L), the MB3 is from 1 to 85,000 μmol/L;
[35]: The methyl methacrylate-containing composition according to any of [31] to [34], wherein in Formula (4) above, $R^{12}$, $R^{13}$, and $R^{14}$ are each independently a hydrogen atom, a $C_{1-5}$ alkyl group, a $C_{2-5}$ alkenyl group, a $C_{1-12}$ aryl group, a $C_{1-6}$ alkoxy group, a $C_{0-6}$ amino group, a $C_{1-6}$ monovalent group containing a carbonyl group, or a $C_{1-5}$ alkylthio group; and $R^{15}$ is a $C_{1-5}$ alkyl group, a $C_{2-5}$ alkenyl group, a $C_{1-12}$ aryl group, a hydroxy group, a $C_{1-6}$ alkoxy group, a $C_{0-6}$ amino group, a $C_{1-6}$ monovalent group containing a carbonyl group, or a $C_{1-5}$ alkylthio group;
[36]: The methyl methacrylate-containing composition according to any of [31] to [35], wherein the concentration of methyl methacrylate is from 99.8 to 99.99% by mass;
[37]: A method of storing a methyl methacrylate-containing composition, in which the methyl methacrylate-containing composition according to any of [1] to [36] is stored at 0 to 50°C;
[38]: A method of producing a methyl methacrylate polymer, comprising a step of polymerizing a polymeric composition comprising a methyl methacrylate-containing composition according to any of [1] to [36]; and
[39]: The method of producing a methyl methacrylate polymer according to [38], wherein the polymeric composition comprises a monomer that can be copolymerized with methyl methacrylate.

EFFECT OF THE INVENTION

According to the present invention, a methyl methacrylate-containing composition having high quality stability with reduced the generation of methyl methacrylate dimer and methyl pyruvate during storage can be provided.

DETAILED DESCRIPTION OF THE INVENTION

Embodiments according to the present invention are described below, but the present invention is not limited to them.
As used herein, any numerical value range indicated by the term "to" means any range including the numerical values described before and after the term "to" as the lower and upper limit values, respectively, and "A to B" means A or more and B or less.

[Methyl Methacrylate-containing Composition 1]

In embodiments of the first aspect, the methyl methacrylate-containing composition comprises methyl methacrylate, a pyrazine compound represented by the following Formula (1) (component A1), and a polymerization inhibitor (component B1), wherein the concentration of methyl methacrylate is from 99 to 99.99% by mass.

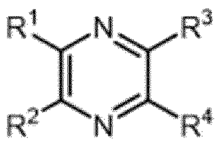

(1)

In Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group.
The methyl methacrylate-containing composition may also contain a component B2 and a component B3 as described later, other compound (component C), and water as long as the composition satisfies a concentration of methyl methacrylate of from 99 to 99.99% by mass. Each item will be described in detail.

(Methyl Methacrylate)

In embodiments of the first aspect, methacrylate-containing composition comprises methyl methacrylate. Methyl methacrylate can be produced, for example, by the acetone cyanohydrin (ACH) method, the new acetone cyanohydrin (new ACH) method, the C4 direct oxidation method, the direct methyl esterification method, the ethylene method, and the new ethylene method. Methyl methacrylate contained in the methyl methacrylate-containing composition is preferably produced by the C4 direct oxidation method, and more preferably produced using biomass-derived isobutanol as starting material by the C4 direct oxidation method.

(Component A1)

In embodiments of the first aspect, the methyl methacrylate-containing composition comprises a pyrazine compound represented by the Formula (1) (component A1). Coexistence of the component A1 and a component B1 described later enables reducing the generation of methyl methacrylate dimers and methyl pyruvate. The reason for this is presumed as follows.

Methyl methacrylate dimers are generated due to radicals generated during storage of methyl methacrylate. Examples of the radicals include hydroxyl radicals generated when oxygen molecules absorb ultraviolet light derived from sunlight. Hydroxyl radicals also cause the generation of methyl pyruvate due to oxidation of methyl methacrylate. The pyrazine compound has an aromatic ring, and thus absorbs ultraviolet light. The absorption wavelength varies depending on the type of the substituent. The pyrazine compound having a structure represented by the Formula (1) can absorb ultraviolet light across a broad wavelength. Therefore, when the methyl methacrylate-containing composition comprises a component A1, a broad wavelength of ultraviolet light is absorbed, and the generation of hydroxyl radicals is reduced. Further inclusion of a component B1 described later enables trapping generated hydroxyl radicals. Therefore, coexistence of the component A1 and the component B1 enables reducing the hydroxyl radical content via two different mechanisms, one in which the component A1 can reduce the generation of hydroxyl radicals, and the other in which the component B1 can remove generated hydroxyl radicals. Thus, it is considered that the generation of methyl methacrylate dimers and methyl pyruvate can be efficiently reduced.

The molecular weight of the component A1 is preferably 1,000 or less. This results in an increased number of pyrazine rings per unit mass in the component A1, so that a less amount of the component A1 can provide the effect of the present invention. The molecular weight of the component A1 is more preferably 800 or less, still more preferably 600 or less, and particularly preferably 400 or less.

In Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group. $R^1$, $R^2$, $R^3$, and $R^4$ may be the same as or different from each other. From the viewpoint of increasing the absorbance of ultraviolet light, and thereby reducing the generation of hydroxyl radicals, $R^1$, $R^2$, $R^3$, and $R^4$ are preferably a hydrogen atom, a $C_{1-5}$ alkyl group, a $C_{2-5}$ alkenyl group, a $C_{1-12}$ aryl group, a $C_{1-6}$ alkoxy group, a $C_{0-6}$ amino group, a $C_{1-6}$ monovalent group containing a carbonyl group, a $C_{1-5}$ alkylthio group, or a $C_{6-10}$ arylthio group, more preferably a hydrogen atom, a $C_{1-5}$ alkyl group, or a $C_{1-6}$ alkoxy group, and still more preferably a hydrogen atom, a methyl group, an ethyl group, an n-propyl group, an isopropyl group, or a methoxy group.

The alkyl group is a chain (linear or branched) alkyl group or a cyclic alkyl group. The alkyl group is preferably a $C_{1-20}$ alkyl group, more preferably a $C_{1-10}$ alkyl group, and still more preferably a $C_{1-5}$ alkyl group. Examples of the chain alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, and an isopropyl group are preferable. Examples of the cyclic alkyl group include a cyclopentyl group, a cyclohexyl group, and a cyclooctyl group.

The alkenyl group is a chain (linear or branched) alkenyl group or a cyclic alkenyl group. The alkenyl group is preferably a $C_{2-20}$ alkenyl group, more preferably a $C_{2-10}$ alkenyl group, and still more preferably a $C_{2-5}$ alkenyl group. Examples of the chain alkenyl group include a vinyl group, a 1-propenyl group, an isopropenyl group, a 2-butenyl group, a 1,3-butadienyl group, a 2-pentenyl group, and a 2-hexenyl group. Examples of the cyclic alkenyl group include a cyclopentenyl group and a cyclohexenyl group.

The aryl group is preferably a $C_{1-20}$ aryl group, and more preferably a $C_{1-12}$ aryl group. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetraazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an

isoindolyl group, an indolyl group, a quinolyl group, a pyridothiazolyl group, a benzimidazolyl group, a benzooxazolyl group, a benzothiazolyl group, a benzotriazolyl group, a benzofuranyl group, an imidazopyridinyl group, a triazopyridinyl group, and a purinyl group.

The alkoxy group is preferably a $C_{1-20}$ alkoxy group, more preferably a $C_{1-10}$ alkoxy group, and still more preferably a $C_{1-6}$ alkoxy group. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group without any substituent on the nitrogen atom ($-NH_2$) ($C_0$), and an amino group in which some or all of the hydrogen atoms bound to the nitrogen atom are substituted with carbon atoms. The number of carbon atoms in an amino group in which some or all of the hydrogen atoms bound to the nitrogen atom are substituted with carbon atoms is preferably from 1 to 20, more preferably from 1 to 10, and still more preferably from 1 to 6. Examples of the amino group include an unsubstituted amino group ($-NH_2$), a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group containing a carbonyl group include a formyl group, an acyl group, a carboxy group, an amide group, an alkoxy carbonyl group, a thiocarboxy group, and a thioester group.

The acyl group is a substituent in which a carbonyl group is linked with an alkyl group, an alkenyl group, or an aryl group. The total number of carbon atoms derived from a carbonyl group (one) and derived from an alkyl group, alkenyl group, or an aryl group is preferably from 2 to 20, more preferably from 2 to 10, and still more preferably from 2 to 6. Examples of the acyl group include an acetyl group, a propionyl group, a butylcarbonyl group, a vinylcarbonyl group, and a benzoyl group.

The amide group includes an amide group without a substituent on the nitrogen atom ($-CONH_2$), and an amide group in which some or all of the hydrogen atoms bound to the nitrogen atom are substituted with carbon atoms. The number of carbon atoms in the amide group, which is the total of the number of carbon atoms derived from a carbonyl group (one) and the number of carbon atoms substituted on the nitrogen atom, is preferably from 1 to 20, more preferably from 1 to 10, and still more preferably from 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxy carbonyl group is a substituent in which a carbonyl group and an alkoxy group are linked, and also referred to as ester group. The total number of carbon atoms derived from a carbonyl group (one) and derived from an alkoxy group is preferably from 2 to 20, more preferably from 2 to 10, and still more preferably from 2 to 6. Examples of the alkoxy carbonyl group include a methoxycarbonyl group, an ethoxycarbonyl group, a butoxycarbonyl group, and a phenoxycarbonyl group.

The thioester group is a substituent in which a carbonyl group and an alkylthio group or an arylthio group are linked. The total number of carbon atoms derived from a carbonyl group (one) and derived from an alkylthio group or an arylthio group is preferably from 2 to 20, more preferably from 2 to 10, and still more preferably from 2 to 6. Examples of the thioester group include a methylthiocarbonyl group, an ethylthiocarbonyl group, a butylthiocarbonyl group, and a phenylthiocarbonyl group.

The monovalent group containing a carbonyl group may be a substituent in which one or more hydrogen(s) in an alkyl group is/are substituted with a carbonyl group(s). Examples of such a substituent include a 2-acetoxyethyl group, a 2-acetoethyl group, and a 2-(acetoacetoxy)ethyl group.

The alkylthio group is preferably a $C_{1-20}$ alkylthio group, more preferably a $C_{1-10}$ alkylthio group, and still more preferably a $C_{1-5}$ alkylthio group. Examples of the alkylthio group include a methylthio group, an ethylthio group, a propylthio group, and an isopropylthio group.

The arylthio group is preferably a $C_{1-20}$ arylthio group, more preferably a $C_{3-10}$ arylthio group, and still more preferably a $C_{6-10}$ arylthio group. Examples of the arylthio group include a phenylthio group, and a tolylthio group.

Among compounds that satisfy the conditions described above, the component A1 is preferably 2,3,5,6-tetramethylpyrazine, pyrazine, 2,3,5-trimethylpyrazine, 2-methoxypyrazine, 2-isopropyl-3-methoxypyrazine, 2,5-dimethylpyrazine, 2,5-diisopropylpyrazine, 2-ethyl-3,5-dimethylpyrazine, 2,5-dimethyl-3-isobutylpyrazine, 2-isopropyl-3-methoxy-5-isobutylpyrazine, 2-aminopyrazine, 2-(methylthio)pyrazine, 2,5-dimethyl-3-(methylthio)pyrazine, 2-pyrazinemethanol, pyrazinemethylamine, methyl 2-pyrazinecarboxylate, 2-vinylpyrazine, or 2-phenylpyrazine, and more preferably 2,3,5,6-tetramethylpyrazine, pyrazine, 2,3,5-trimethylpyrazine, 2-methoxypyrazine, 2-isopropyl-3-methoxypyrazine, or 2,5-dimethylpyrazine, from the viewpoint of convenient availability and synthesis.

In embodiments of the first aspect, one or two or more component(s) A1 may be contained in the methyl methacrylate-containing composition.

(Component B1)

In embodiments of the first aspect, the methyl methacrylate-containing composition comprises a polymerization inhibitor (Component B1). As used herein, the term "polymerization inhibitor" means a compound having a function to inhibit the polymerization reaction of methyl methacrylate. Examples of the polymerization inhibitor include a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound. Inclusion of the component B1 enables reducing the progress of the polymerization reaction of methyl methacrylate via a radical polymerization mechanism during storage of methyl methacrylate. The component B1 can also trap hydroxyl radicals as described above generated during storage of methyl methacrylate. That is, when the methyl methacrylate-containing composition comprises a component A1 as well as a component B1, the hydroxyl radical content can be reduced via two different mechanisms, one in which the component A1 can reduce the generation of hydroxyl radicals, and the other in which the component B1 can remove generated hydroxyl radicals. Thus, it is considered that the generation of methyl methacrylate dimers and methyl pyruvate can be efficiently reduced.

Examples of the polymerization inhibitor that is a phenol compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylene-bis(6-t-butyl-4-methylphenol), 2,2'-methylene-bis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, dit-butyl-4-methoxyphenol, pyrogallol-1,2-dimethylether, and hydroquinone monobenzoate.

Examples of the tocopherol include $\alpha$-tocopherol, and 2,3-dihydro-2,2-dimethyl-7-hydroxybenzofuran.

Examples of the polymerization inhibitor that is a quinone compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor that is a nitrobenzene compound include nitrobenzene, o-dinitrobenzene, m-dinitrobenzene, p-dinitrobenzene, 2,4-dinitrotoluene, dinitrodurene, and 2,2-diphenyl-1-picrylhydrazyl.

Examples of the polymerization inhibitor that is an N-oxyl compound include 4-hydroxy-2,2,6,6-tetramethylpiperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethanoloxy-2,2,6,6-tetramethyl-piperidine-N-oxyl, 4-benzoyloxy-2,2,6,6-tetramethylpiperidine-N-oxyl, 2,2,5,5-tetramethyl-piperidine-N-oxyl, 3-amino-2,2,5,5-tetramethyl-piperidine-N-oxyl, 4,4',4-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butylnitroxide, and di-tert-amylnitroxide.

Examples of the polymerization inhibitor that is an amine compound include N,N-diphenylamine, alkylated diphenylamine, 4,4'-dicumyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (the alkyl groups may be the same or different, and may each independently comprise 1 to 4 carbon atoms, and may be linear or branched-chain), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-$\alpha$-naphthylamine, N-phenyl-$\beta$-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor that is a phosphorus-containing compound include triphenylphosphine, triphenyl phosphite, triethyl phosphite, tris(isodecyl) phosphite, tris(tridecyl) phosphite, phenyl diisooctyl phosphite, phenyl diisodecyl phosphite, phenyl di(tridecyl) phosphite, diphenyl isooctyl phosphite, diphenyl isodecyl phosphite, diphenyl tridecyl phosphite, phosphonic acid [1,1-diphenyl-4,4'-diylbistetrakis-2,4-bis(1,1-dimethylethyl)phenyl] ester, tris(nonylphenyl) phosphite, 4,4'-isopropylidenediphenol alkyl phosphite, tris(2,4-di-tert-butylphenyl) phosphite, tris(biphenyl) phosphite, distearyl pentaerythritol diphosphite, di(2,4-di-tert-butylphenyl) pentaerythritol diphosphate, di(nonylphenyl) pentaerythritol diphosphite, phenyl bisphenol-A pentaerythritol diphosphite, tetra(tridecyl)-4,4'-butylidenebis(3-methyl-6-tert-butylphenol)diphosphite, hexa(tridecyl)-1,1,3-tris(2-methyl-4-hydroxy-5-tert-butylphenyl) butanetriphosphite, 3,5-di-tert-butyl-4-hydroxybenzyl phosphate diethyl ester, sodium-bis(4-tert-butylphenyl) phosphate, sodium-2,2'-methylene -bis(4,6-di-tert-butylphenyl)phosphate, and 1,3-bis(diphenoxyphosphonyloxy)benzene.

Examples of the polymerization inhibitor that is a sulfur-containing compound include diphenyl sulfide, phenothiazine, 3-oxophenothiazine, 5-oxophenothiazine, phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor that is an iron-containing compound include iron (III) chloride.

Examples of the polymerization inhibitor that is a copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor that is a manganese-containing compound include manganese dialkyl dithiocarbamate (the alkyl groups are each any of a methyl group, an ethyl group, a propyl group, and a butyl group, and may be the same or different), manganese diphenyl dithiocarbamate, manganese formate, manganese acetate, manganese octanoate, manganese naphthenate, manganese permanganate, and ethylenediaminetetraacetic acid manganese salt.

Among those described above, the component B1 is preferably at least one polymerization inhibitor(s) selected from the group consisting of a phenol compound, an N-oxyl compound, an amine compound, a phosphorus-containing compound, and a sulfur-containing compound, more preferably at least one polymerization inhibitor(s) selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine, and still more preferably at least one polymerization inhibitor(s) selected from the group consisting of hydroquinone, 4-methoxyphenol, 2,4-dimethyl-6-t-butylphenol, N,N-diphenylamine, triphenyl phosphite, and phenothiazine, from the viewpoint of quality stability of the methyl methacrylate-containing composition during storage.

One or two or more component(s) B1 may be contained.

In cases where the methyl methacrylate-containing composition comprises a compound that corresponds to both components A1 and B1, the compound is considered as the component B1. Thus, the methyl methacrylate-containing composition must comprise a component A1 other than the compound. In cases where two or more compounds that correspond to both components A1 and B1 are contained, the compound with the highest molar concentration in the methyl methacrylate-containing composition is considered as the component B1, and the other compound(s) is/are considered as the component(s) A1.

(Concentrations of Component A1 and Component B1)

When the concentration of the component A1 is MA1 (umol/L) and the concentration of the component B1 is MB1 (umol/L), the MB1/MA1 is preferably 0.001 or more, and more preferably 0.005 or more from the viewpoint of efficiency in reducing the generation of methyl pyruvate. The upper limit of the MB1/MA1 is preferably 100 or less, more preferably 50 or less, still more preferably 30 or less, and particularly preferably 10 or less.

The MA1 is preferably from 1 to 65,000 μmol/L. When the MA1 is 1 μmol/L or more, the effect of reducing the generation of methyl methacrylate dimer and methyl pyruvate can be sufficiently obtained. When the MA1 is 65,000 μmol/L or less, the impurity content after the production of methyl methacrylate polymer by polymerization of the methyl methacrylate-containing composition according to the present embodiment is reduced, so that adverse effects on physical properties of the polymer can be prevented. The lower limit of the MA1 is more preferably 10 μmol/L or more, and still more preferably 50 μmol/L or more. The upper limit of the MA1 is more preferably 30,000 μmol/L or less, and still more preferably 10,000 μmol/L or less.

The MB1 is preferably from 1 to 7,000 μmol/L. When the MB1 is 1 μmol/L or more, the effect of reducing the generation of methyl methacrylate dimer and methyl pyruvate can be sufficiently obtained. When the MB1 is 7,000 μmol/L or less, the impurity content after the production of methyl methacrylate polymer by polymerization of the methyl methacrylate-containing composition according to the present embodiment is reduced, so that adverse effects on physical

properties of the polymer can be prevented. The lower limit of the MB1 is more preferably 10 μmol/L or more, and still more preferably 40 μmol/L or more. The upper limit of the MB1 is more preferably 5,000 mol/L or less, still more preferably 1,000 μmol/L or less, and particularly preferably 600 μmol/L or less.

(Concentration of Methyl Methacrylate)

In embodiments of the first aspect, the concentration of methyl methacrylate in the methyl methacrylate-containing composition is from 99 to 99.99% by mass. When the concentration of methyl methacrylate is 99% by mass or more, the impurity content after the production of methyl methacrylate polymer by polymerization of the methyl methacrylate-containing composition is reduced, so that adverse effects on the physical properties of the polymer can be prevented. When the concentration of methyl methacrylate is 99.99% by mass or less, the purification cost can be reduced. The lower limit of the concentration of methyl methacrylate is preferably 99.8% by mass or more.

(Component C)

In embodiments of the first aspect, the methyl methacrylate-containing composition may contain other compound (component C) as long as the composition satisfies a concentration of methyl methacrylate of from 99 to 99.99% by mass. Examples of the component C include impurities generated during the production of methyl methacrylate. For example, methyl methacrylate may contain diacetyl as an impurity. From the viewpoint of reducing coloration of the methyl methacrylate-containing composition, the concentration of diacetyl is preferably 55 μmol/L or less, more preferably 20 μmol/L or less, still more preferably 10 μmol/L or less, and particularly preferably 1 μmol/L.

(Analysis of Methyl Methacrylate-containing Composition)

Inclusion of a component A1, a component B1, a component B2 and a component B3 as described later, a component C, and water by the methyl methacrylate-containing composition can be determined, for example, by GC-MS measurement. When the GC-MS chart of the methyl methacrylate-containing composition has a peak at the same retention time as a reference material for the component A1, and when the m/z value detected in the mass spectrum of the peak corresponds to the mass of the component A1, it can be determined that the methyl methacrylate-containing composition contains the component A1. In cases where the reference material of the component A1 is not available, it can be determined that the peak is the component A1 peak when the mass spectrum peak pattern shown in the GC-MS chart of the methyl methacrylate-containing composition corresponds to the mass spectrum pattern of the component A1 recorded in the mass spectrum database. In other words, it can be determined that the methyl methacrylate-containing composition comprises the component A1. Examples of the mass spectrum database include NIST20, NIST17, NIST14, and NIST14s. When detection by GC-MS measurement is impossible due to low volatility (the boiling point is 500°C or higher), LC-MS can be used for detection. Inclusion of a component B1, a component B2 and a component B3 as described later, a component C, and water can also be determined by the same method.

The concentration of methyl methacrylate can be determined by performing GC-FID measurement of the methyl methacrylate-containing composition, quantifying methyl methacrylate using the area normalization method, and correcting the resulting value using the water concentration determined with a Karl-Fisher moisture meter. The concentration of the component A1 can be determined by performing GC measurement of the methyl methacrylate-containing composition and using the internal standard method. When the reference material of the component A1 is not available, and thus cannot be quantified by the internal standard method, GC-FID measurement for any organic compound having known concentration is performed under the same conditions as the methacrylate-containing composition, and then the concentration of the component A1 can be calculated using the following equation:

$$\text{The concentration of the component A1 } (\mu\text{mol/L}) = \frac{N}{N_{A1}} \times \frac{S_{A1}}{S} \times \text{M}$$

In the equation, N is the number of carbon atoms that an organic compound having known concentration contains in one molecule; $N_{A1}$ is the number of carbon atoms that the component A1 contains in one molecule; $S_{A1}$ is the peak area of the component A1, S is the peak area of an organic compound having known concentration, and M is the concentration (μmol/L) of an organic compound having known concentration.

When quantification by GC measurement is impossible due to low volatility (the boiling point is 500°C or higher), LC measurement can be used for quantification.

The concentrations of the component B1, component B2, component B3, and component C can be also determined by the same method as the component A1 described above.

Inclusion of water by the methacrylate-containing composition and its concentration can be determined by the Karl-Fischer method.

[Methyl Methacrylate-containing Composition 2]

In embodiments of the second aspect, the methyl methacrylate-containing composition comprises methyl methacrylate, and a pyrazine compound represented by the following Formula (2) (component A2), wherein the concentration of methyl methacrylate is from 99 to 99.99% by mass.

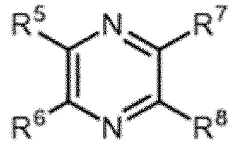 (2)

In Formula (2) above, $R^5$, $R^6$, $R^7$, and $R^8$ each independently represent a hydrogen atom, an alkyl group, an alkenyl group having a carbon number of 3 or more, an aryl group, a hydroxy group, an alkoxy group, an amino group, a monovalent group containing a carbonyl group, an alkylthio group, or an arylthio group.
The methyl methacrylate-containing composition may also contain the component B1, a component B2 and a component B3 as described later, other compound (component C), and water as long as the composition satisfies a concentration of methyl methacrylate of from 99 to 99.99% by mass. Each item will be described in detail.

(Methyl Methacrylate)

In embodiments of the second aspect, methacrylate-containing composition comprises methyl methacrylate. In preferred aspects, methyl methacrylate is the same as the first aspect.

(Component A2)

In embodiments of the second aspect, the methyl methacrylate-containing composition comprises a pyrazine compound represented by Formula (2) (component A2). When the methyl methacrylate-containing composition comprises a component A2, the component A2 can intensely absorb ultraviolet light with a wavelength that causes the generation of hydroxyl radicals and sufficiently reduce the generation of hydroxyl radicals. Thus, even in cases where a component B1 for trapping generated hydroxyl radicals is not present, the generation of methyl methacrylate dimers and methyl pyruvate can be reduced. However, the methyl methacrylate-containing composition in the second aspect preferably comprises a component B1 as described above from the viewpoint that the generation of methyl methacrylate dimers and methyl pyruvate can be reduced at a higher level.
The molecular weight of the component A2 is preferably 1,000 or less. This results in an increased number of pyrazine rings per unit mass in the component A2, so that a less amount of the component A2 can provide the effect of the present invention. The molecular weight of the component A2 is more preferably 800 or less, still more preferably 600 or less, and particularly preferably 400 or less.
In the Formula (2), $R^5$, $R^6$, $R^7$, and $R^8$ each independently represent a hydrogen atom, an alkyl group, an alkenyl group having a carbon number of 3 or more, an aryl group, a hydroxy group, an alkoxy group, an amino group, a monovalent group containing a carbonyl group, an alkylthio group, or an arylthio group. $R^5$, $R^6$, $R^7$, and $R^8$ may be the same as or different from each other. From the viewpoint of increasing the absorbance of ultraviolet light, and thereby reducing the generation of hydroxyl radicals, $R^5$, $R^6$, $R^7$, and $R^8$ are preferably a hydrogen atom, a $C_{1-5}$ alkyl group, a $C_{3-5}$ alkenyl group, a $C_{1-12}$ aryl group, a $C_{1-6}$ alkoxy group, a $C_{0-6}$ amino group, a $C_{1-6}$ monovalent group containing a carbonyl group, a $C_{1-5}$ alkylthio group, or a $C_{6-10}$ arylthio group, more preferably a hydrogen atom, a $C_{1-5}$ alkyl group, or a $C_{1-6}$ alkoxy group, and still more preferably a hydrogen atom, a methyl group, an ethyl group, an n-propyl group, an isopropyl group, or a methoxy group.
In preferred embodiments, the alkyl group, the aryl group, the alkoxy group, the amino group, the monovalent group containing a carbonyl group, the alkylthio group, and the arylthio group in the Formula (2) are the same as Formula (1) above.
The alkenyl group is a chain (linear or branched) alkenyl group or a cyclic alkenyl group having a carbon number of 3 or more. The alkenyl group is preferably a $C_{3-20}$ alkenyl group, more preferably a $C_{3-10}$ alkenyl group, and still more preferably a $C_{3-5}$ alkenyl group. Examples of the chain alkenyl group include a 1-propenyl group, an isopropenyl group, a 2-butenyl group, a 1,3-butadienyl group, a 2-pentenyl group, and a 2-hexenyl group. Examples of the cyclic alkenyl group include a cyclopentenyl group and a cyclohexenyl group.

Among compounds that satisfy the conditions described above, the component A2 is preferably 2,3,5,6-tetramethylpyrazine, pyrazine, 2,3,5-trimethylpyrazine, 2-methoxypyrazine, 2-isopropyl-3-methoxypyrazine, 2,5-dimethylpyrazine, 2,5-diisopropylpyrazine, 2-ethyl-3,5-dimethylpyrazine, 2,5-dimethyl-3-isobutylpyrazine, 2-isopropyl-3-methoxy-5-isobutylpyrazine, 2-aminopyrazine, 2-(methylthio)pyrazine, 2,5-dimethyl-3-(methylthio)pyrazine, 2-pyrazinemethanol, pyrazinemethylamine, methyl 2-pyrazinecarboxylate, or 2-phenylpyrazine, and more preferably 2,3,5,6-tetramethylpyrazine, pyrazine, 2,3,5-trimethylpyrazine, 2-methoxypyrazine, 2-isopropyl-3-methoxypyrazine, or 2,5-dimethylpyrazine, from the viewpoint of convenient availability and synthesis.

In embodiments of the second aspect, one or two or more component(s) A2 may be contained in the methyl methacrylate-containing composition.

(Component B1)

In preferred aspects where the methyl methacrylate-containing composition comprises a component B1, the component B1 is the same as the first aspect. One or two or more component(s) B1 may be contained.

In cases where the methyl methacrylate-containing composition comprises a compound that corresponds to both components A2 and B1, the compound is considered as the component A2. Thus, the case where the methyl methacrylate-containing composition comprises a component A2 and a component B1 means that the composition comprises the component B1 other than the compound. In cases where two or more compounds that correspond to both components A2 and B1 are contained, the compound with the highest molar concentration in the methyl methacrylate-containing composition is considered as the component A2, and the other compound(s) is/are considered as the component(s) B1.

(Concentrations of Component A2 and Component B1)

When the concentration of the component A2 is MA2 (umol/L), the MB1/MA2 is preferably 0.001 or more, and more preferably 0.005 or more from the viewpoint of efficiency in reducing the generation of methyl pyruvate. The upper limit of the MB1/MA2 is preferably 100 or less, more preferably 50 or less, still more preferably 30 or less, and particularly preferably 10 or less.

The MA2 is preferably from 1 to 65,000 μmol/L. When the MA2 is 1 μmol/L or more, the effect of reducing the generation of methyl methacrylate dimer and methyl pyruvate can be sufficiently obtained. When the MA2 is 65,000 μmol/L or less, the impurity content after the production of methyl methacrylate polymer by polymerization of the methyl methacrylate-containing composition according to the present embodiment is reduced, so that adverse effects on physical properties of the polymer can be prevented. The lower limit of the MA2 is more preferably 10 μmol/L or more, and still more preferably 50 μmol/L or more. The upper limit of the MA2 is more preferably 30,000 μmol/L or less, and still more preferably 10,000 μmol/L or less.

In preferred aspects, the MB1 is the same as the first aspect.

(Concentration of Methyl Methacrylate)

In preferred aspects, the concentration of methyl methacrylate is the same as the first aspect.

(Component C)

In preferred aspects, the component C is the same as the first aspect.

(Analysis of Methyl Methacrylate-containing Composition)

The method of confirming that the methyl methacrylate-containing composition contains a component A2, a component B1, a component C, and water, and the method of measuring the concentrations of methyl methacrylate, a component A2, a component B1, a component C, and water are the same as the first aspect.

[Methyl Methacrylate-containing Composition 3]

In embodiments of the third aspect, the methyl methacrylate-containing composition comprises methyl methacrylate, a pyrazine compound represented by the following Formula (1) (component A1), and an ester compound having an α-hydrogen represented by the following Formula (3) (component B2), wherein the concentration of methyl methacrylate is from 99 to 99.99% by mass.

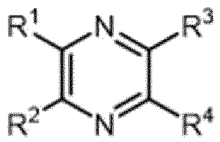

(1)

In Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group.

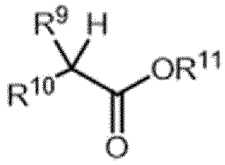

(3)

In Formula (3) above, $R^9$ and $R^{10}$ each independently represent a hydrogen atom, an alkyl group, an alkenyl group, a hydroxy group, an alkoxy group, an amino group, a monovalent group containing a carbonyl group, or an alkylthio group. $R^{11}$ represents an alkyl group or an aryl group. $R^9$ and $R^{10}$, $R^{10}$ and $R^{11}$, and $R^{11}$ and $R^9$ each may be linked to each other to form a ring.

The methyl methacrylate-containing composition may also contain the component B1, a component B3 as described later, other compound (component C), and water as long as the composition satisfies a concentration of methyl methacrylate of from 99 to 99.99% by mass. Each item will be described in detail.

(Methyl Methacrylate)

In embodiments of the third aspect, methacrylate-containing composition comprises methyl methacrylate. In preferred aspects, methyl methacrylate is the same as the first aspect.

(Component A1)

In embodiments of the third aspect, the methyl methacrylate-containing composition comprises a pyrazine compound represented by Formula (1) above (component A1). Coexistence of the component A1 and a component B2 described later enables reducing the generation of methyl methacrylate dimers and methyl pyruvate. The reason for this is presumed as follows.

As described above, since methyl methacrylate dimers are mainly generated due to hydroxyl radicals generated during storage of methyl methacrylate, and the component A1 reduces the generation of hydroxyl radicals, the component A1 can reduce the generation of methyl methacrylate dimers. However, the dimerization reaction of methyl methacrylate also proceeds by an anionic mechanism under a basic condition. An ester compound having an $\alpha$-hydrogen is weak acid and can trap anions. Thus, the component B2 can reduce the dimerization reaction of methyl methacrylate by an anionic mechanism. Thus, it is considered that coexistence of the component A1 and the component B2 enables efficiently reducing the dimerization reaction of methyl methacrylate via different mechanisms.

On the other hand, methyl pyruvate is generated by oxidation of methyl methacrylate with hydroxy radicals and oxygen molecules as described above. The component A1 can reduce the generation of hydroxy radicals, while the component B2 can trap a radical intermediate generated by the reaction of a hydroxy radical and methyl methacrylate and convert the intermediate back to methyl methacrylate. Thus, it is considered that coexistence of the component A1 and the component B2 enables efficiently reducing the generation of methyl pyruvate.

In preferred aspects, the component A1 is the same as the first aspect.

(Component B2)

In embodiments of the third aspect, the methyl methacrylate-containing composition comprises an ester compound having an $\alpha$-hydrogen represented by Formula (3) above (component B2). The term "a-hydrogen" represents a hydrogen atom bound to a carbon atom next to a carbon atom of a carbonyl group.

The molecular weight of the component B2 is preferably 1,000 or less. When the molecular weight is 1,000 or less, the number of $\alpha$-hydrogens per unit mass in the component B2 can be increased, so that the effect of the present invention can be obtained with less mass. The molecular weight of the component B2 is more preferably 800 or less,

still more preferably 600 or less, and particularly preferably 400 or less.

In the Formula (3), $R^9$ and $R^{10}$ each independently represent a hydrogen atom, an alkyl group, an alkenyl group, a hydroxy group, an alkoxy group, an amino group, a monovalent group containing a carbonyl group, or an alkylthio group. $R^9$ and $R^{10}$ may be the same or different. $R^{11}$ in the Formula (3) represents an alkyl group or an aryl group. $R^{11}$ and $R^9$, and $R^{11}$ and $R^{10}$ may be the same or different. $R^9$ and $R^{10}$, $R^{10}$ and $R^{11}$, and $R^{11}$ and $R^9$ each may be linked to each other to form a ring.

In general, an $\alpha$-hydrogen in an ester compound is reactive with an anion or a radical, and may have reduced reactivity depending on the type of the substituent that possesses the $\alpha$-hydrogen. In cases where $R^9$, $R^{10}$, and $R^{11}$ satisfy the conditions described above, the reactivity of the $\alpha$-hydrogen of the component B2 with an anion or a radical is maintained, so that the effect of the present invention can be obtained. $R^9$ and $R^{10}$ each are preferably a hydrogen atom, a $C_{1-5}$ alkyl group, a hydroxy group, a $C_{1-6}$ alkoxy group, a $C_{0-6}$ amino group, a $C_{1-6}$ monovalent group containing a carbonyl group, or a $C_{1-5}$ alkylthio group. They are highly stable substituents, thus allowing for prevention of the component B2 from being changed into other compounds during storage. In addition, they also have less electron-donating properties, so that the acidic properties of the $\alpha$-hydrogen of the component B2 is increased. $R^9$ and $R^{10}$ each are more preferably a hydrogen atom or a $C_{1-5}$ alkyl group, and still more preferably a hydrogen atom, a methyl group, an ethyl group, an n-propyl group, or an isopropyl group. $R^{11}$ is more preferably a $C_{1-5}$ alkyl group or a $C_{1-12}$ aryl group. They are highly stable substituents, thus allowing for prevention of the component B2 from being changed into other compounds during storage. $R^{11}$ is more preferably a $C_{1-5}$ alkyl group, and still more preferably a methyl group.

In preferred embodiments, the alkyl group, the alkenyl group, the alkoxy group, the amino group, the monovalent group containing a carbonyl group, the alkylthio group, and the aryl group are the same as Formula (1) above.

$R^9$ and $R^{10}$, $R^{10}$ and $R^{11}$, and $R^{11}$ and $R^9$ each may be linked to each other to form a ring. Examples of the compound in which $R^9$ and $R^{10}$ are linked to form a ring include methyl cyclohexanecarboxylate, and methyl cyclopentanecarboxylate. Examples of the compound in which $R^{10}$ and $R^{11}$ are linked to form a ring, and the compound in which $R^{11}$ and $R^9$ are linked to form a ring include $\alpha$-methyl-$\delta$-valerolactone, and $\alpha$-methyl-$\gamma$-butyrolactone.

Among compounds that satisfy the conditions described above, the component B2 is preferably methyl isobutyrate, methyl propionate, isobutyl isobutyrate, methyl isovalerate, methyl 2-methylbutyrate, isoamyl isobutyrate, methyl lactate, methyl 2-methoxypropionate, N,N-dimethylglycine methyl, dimethyl malonate, methyl (methylthio)acetate, methyl 3-butenoate, methyl (R)-(-)-3-hydroxyisobutyrate, methyl acetate, ethyl acetate, phenyl acetate, ethyl propionate, ethyl isobutyrate, phenyl isobutyrate, methyl butyrate, methyl cyclohexanecarboxylate, methyl cyclopentanecarboxylate, $\alpha$-methyl-$\delta$-valerolactone, or $\alpha$-methyl-$\gamma$-butyrolactone; more preferably methyl isobutyrate, methyl propionate, isobutyl isobutyrate, methyl isovalerate, methyl 2-methylbutyrate, isoamyl isobutyrate, methyl lactate, N,N-dimethylglycine methyl, dimethyl malonate, methylthiomethyl acetate, methyl 3-butenoate, methyl (R)-(-)-3-hydroxyisobutyrate, or methyl cyclohexanecarboxylate; and still more preferably methyl isobutyrate, methyl propionate, isobutyl isobutyrate, or methyl 2-methylbutyrate, from the viewpoint of quality stability of methyl methacrylate-containing composition during storage.

In embodiments of the third aspect, one or two or more component(s) B2 may be contained in the methyl methacrylate-containing composition.

In cases where the methyl methacrylate-containing composition comprises a compound that corresponds to both components A1 and B2, the compound is considered as the component A1. Thus, the methyl methacrylate-containing composition must comprise a component B2 other than the compound. In cases where two or more compounds that correspond to both components A1 and B2 are contained, the compound with the highest molar concentration in the methyl methacrylate-containing composition is considered as the component A1, and the other compound(s) is/are considered as the component(s) B2.

(Concentrations of Component A1 and Component B2)

When the concentration of the component B2 is MB2 (umol/L), the MB2/MA1 is preferably 0.01 or more, more preferably 0.1 or more, and still more preferably 0.5 or more from the viewpoint of efficiency in reducing the generation of methyl pyruvate. The upper limit of the MB2/MA1 is preferably 1,000 or less, more preferably 500 or less, still more preferably 100 or less, particularly preferably 50 or less, and most preferably 10 or less.

In preferred aspects, the MA1 is the same as the first aspect.

The MB2 is preferably from 1 to 50,000 $\mu$mol/L. When MB2 is 1 $\mu$mol/L or more, the effect of reducing the generation of methyl methacrylate dimer and methyl pyruvate can be sufficiently obtained. When the MB2 is 50,000 $\mu$mol/L or less, the impurity content after the production of methyl methacrylate polymer by polymerization of the methyl methacrylate-containing composition according to the present embodiment is reduced, so that adverse effects on physical properties of the polymer can be prevented. The lower limit of MB2 is more preferably 10 $\mu$mol/L or more, and still more preferably 50 $\mu$mol/L or more. The upper limit of the MB2 is more preferably 30,000 $\mu$mol/L or less, and still more preferably 10,000 $\mu$mol/L or less.

(Concentration of Methyl Methacrylate)

In preferred aspects, the concentration of methyl methacrylate is the same as the first aspect.

(Component C)

In preferred aspects, the component C is the same as the first aspect.

(Analysis of Methyl Methacrylate-containing Composition)

The method of confirming that the methyl methacrylate-containing composition contains a component A1, a component B2, a component C, and water, and the method of measuring the concentrations of methyl methacrylate, a component A1, a component B2, a component C, and water are the same as the first aspect.

[Methyl Methacrylate-containing Composition 4]

In embodiments of the fourth aspect, the methyl methacrylate-containing composition comprises methyl methacrylate, a pyrazine compound represented by the following Formula (1) (component A1), and an α,β-unsaturated carbonyl compound represented by the following Formula (4) (component B3), wherein the concentration of methyl methacrylate is from 99 to 99.99% by mass.

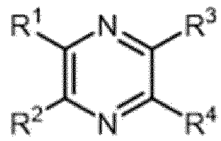

(1)

In Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group.

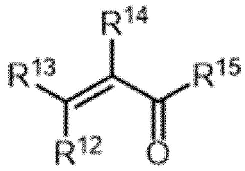

(4)

In Formula (4) above, $R^{12}$, $R^{13}$, and $R^{14}$ each independently represent a hydrogen atom, an alkyl group, an alkenyl group, an aryl group, a hydroxy group, an alkoxy group, an amino group, a monovalent group containing a carbonyl group, an alkylthio group, or an arylthio group. $R^{15}$ represents an alkyl group, an alkenyl group, an aryl group, a hydroxy group, an alkoxy group, an amino group, a monovalent group containing a carbonyl group, an alkylthio group, or an arylthio group. $R^{12}$ and $R^{13}$, $R^{13}$ and $R^{14}$, and $R^{14}$ and $R^{15}$ each may be linked to each other to form a ring, except when $R^{12}$ = H, $R^{13}$ = H, $R^{14}$ = $CH_3$, and $R^{15}$ = $OCH_3$, that is, when the α,β-unsaturated carbonyl compound represented by Formula (4) is methyl methacrylate. H represents a hydrogen atom, C represents a carbon atom, and O represents an oxygen atom.

The methyl methacrylate-containing composition may also contain the component B1 and the component B2, other compound (component C), and water as long as the composition satisfies a concentration of methyl methacrylate of from 99 to 99.99% by mass. Each item will be described in detail.

(Methyl Methacrylate)

In embodiments of the fourth aspect, methacrylate-containing composition comprises methyl methacrylate. In preferred aspects, methyl methacrylate is the same as the first aspect.

(Component A1)

In embodiments of the fourth aspect, the methyl methacrylate-containing composition comprises a pyrazine

compound represented by Formula (1) above (component A1). Coexistence of the component A1 and a component B3 described later enables reducing the generation of methyl methacrylate dimers and methyl pyruvate. The reason for this is presumed as follows.

As described above, since methyl methacrylate dimers and methyl pyruvate are generated due to hydroxyl radicals generated during storage of methyl methacrylate, and the component A1 absorbs ultraviolet light to reduce the generation of hydroxyl radicals, the component A1 can reduce the generation of methyl methacrylate dimers. On the other hand, the $\alpha,\beta$-unsaturated carbonyl compound represented by Formula (4) above (component B3) also has a conjugated double bond and thus absorbs ultraviolet light, but has an absorption wavelength different from that of the component A1. Thus, it is considered that coexistence of the component A1 and the component B3 enables absorbing ultraviolet light across a broad wavelength and efficiently reducing the generation of hydroxyl radicals.

In preferred aspects, the component A1 is the same as the first aspect.

(Component B3)

In embodiments of the fourth aspect, the methyl methacrylate-containing composition comprises an $\alpha,\beta$-unsaturated carbonyl compound represented by Formula (4) above (component B3).

The molecular weight of the component B3 is preferably 1,000 or less. When the molecular weight is 1,000 or less, the number of conjugated double bonds per unit mass in the component B3 can be increased, so that the effect of the present invention can be obtained with less mass. The molecular weight of the component B3 is more preferably 800 or less, still more preferably 600 or less, and particularly preferably 400 or less.

In the Formula (4), $R^{12}$, $R^{13}$, and $R^{14}$ each independently represent a hydrogen atom, an alkyl group, an alkenyl group, an aryl group, a hydroxy group, an alkoxy group, an amino group, a monovalent group containing a carbonyl group, an alkylthio group, or an arylthio group. $R^{15}$ in the Formula (4) represents an alkyl group, an alkenyl group, an aryl group, a hydroxy group, an alkoxy group, an amino group, a monovalent group containing a carbonyl group, an alkylthio group, or an arylthio group. $R^{12}$, $R^{13}$, $R^{14}$, and $R^{15}$ may be the same as or different from each other.

When $R^{12}$, $R^{13}$, $R^{14}$, and $R^{15}$ satisfy the conditions described above, the n-conjugated system in the component B3 is maintained, and thus the component B3 has a property of absorbing a broad wavelength of ultraviolet light, so that the effect of the present invention can be obtained. $R^{12}$, $R^{13}$, and $R^{14}$ each are preferably a hydrogen atom, a $C_{1-5}$ alkyl group, a $C_{2-5}$ alkenyl group, a $C_{1-12}$ aryl group, a $C_{1-6}$ alkoxy group, a $C_{0-6}$ amino group, a $C_{1-6}$ monovalent group containing a carbonyl group, or a $C_{1-5}$ alkylthio group. They are highly stable substituents, thus allowing for prevention of the component B3 from being changed into other compounds during storage. When the component B3 has such a substituent, a sufficient amount of ultraviolet light can be absorbed by the component B3 as a single molecule. $R^{12}$, $R^{13}$, and $R^{14}$ are more preferably a hydrogen atom or a $C_{1-5}$ alkyl group, and still more preferably a hydrogen atom, a methyl group, an ethyl group, an n-propyl group, or an isopropyl group.

$R^{15}$ is preferably a $C_{1-5}$ alkyl group, a $C_{2-5}$ alkenyl group, a $C_{1-12}$ aryl group, a hydroxy group, a $C_{1-6}$ alkoxy group, a $C_{0-6}$ amino group, a $C_{1-6}$ monovalent group containing a carbonyl group, or a $C_{1-5}$ alkylthio group. They are highly stable substituents, thus allowing for prevention of the component A1 from being changed into other compounds during storage. $R^{15}$ is more preferably a $C_{1-5}$ alkyl group or a $C_{1-5}$ alkoxy group, and still more preferably a methyl group, a methoxy group, an ethoxy group, an n-propoxy group, an isopropoxy group, an n-butoxy group, an isobutoxy group, or an isopentoxy group. When $R^{15}$ is the structure described above, the methyl methacrylate-containing composition can have higher quality stability during storage.

In preferred embodiments, the alkyl group, the alkenyl group, the aryl group, the alkoxy group, the amino group, the monovalent group containing a carbonyl group, the alkylthio group, and the arylthio group are the same as Formula (1) above.

$R^{12}$ and $R^{13}$, $R^{13}$ and $R^{14}$, and $R^{14}$ and $R^{15}$ each may be linked to each other to form a ring. Examples of the compound in which $R^{12}$ and $R^{13}$ are linked to form a ring include methyl 2-cyclohexylidenepropionate, and methyl 2-cyclopentylidenepropionate. Examples of the compound in which $R^{13}$ and $R^{14}$ are linked to form a ring include methyl 1-cyclohexene-1-carboxylate, and methyl 1-cyclopentene-1-carboxylate. Examples of the compound in which $R^{14}$ and $R^{15}$ are linked to form a ring include $\alpha$-methylene-$\delta$-valerolactone, and $\alpha$-methylene-$\gamma$-butyrolactone.

Among compounds that satisfy the conditions described above, the component B3 is preferably methyl acrylate, butyl acrylate, ethyl methacrylate, methyl crotonate, cis-methyl crotonate, isobutyl methacrylate, butyl methacrylate, propyl methacrylate, isopentyl methacrylate, methyl 2-methylene-3-butenoate, methyl 3,3-dimethylacrylate, methyl 2-ethylacrylate, methyl 2-pentenoate, methyl cinnamate, methyl 3-methoxyacrylate, dimethyl fumarate, methacrylamide, acrylic acid, methacrylic acid, crotonic acid, cis-crotonic acid, 3,3-dimethylacrylic acid, 2-ethylacrylic acid, 2-methylene-3-butenoic acid, trans-3-hexen-2-one, isopropenyl methyl ketone, methyl 2-cyclohexylidenepropionate, methyl 2-cyclopentylidenepropionate, methyl 1-cyclohexene-1-carboxylate, methyl 1-cyclopentene-1-carboxylate, $\alpha$-methylene -$\delta$-valerolactone, $\alpha$-methylene-$\gamma$-butyrolactone, or isopropenyl methyl ketone, more preferably methyl acrylate, butyl acrylate, ethyl methacrylate, methyl crotonate, methyl cis-crotonate, isobutyl methacrylate, butyl methacrylate, propyl methacrylate, isopentyl methacrylate, methyl 2-methylene-3-butenoate, methyl 3,3-dimethylacrylate, methyl 2-ethylacrylate, methyl 2-pentenoate, methyl cinnamate, methyl 3-methoxyacrylate, dimethyl fumarate, methyl 1-cyclohexene-1-carboxylate, methacrylamide, acrylic acid, methacrylic acid, crotonic acid, cis-crotonic acid, 3,3-dimethylacrylic acid, 2-ethylacrylic acid, 2-methylene -3-butenoic acid, trans-3-hexen-2-one, or isopropenyl methyl ketone, and still more preferably methyl acrylate, ethyl methacrylate, methyl crotonate, isobutyl methacrylate, butyl methacrylate, propyl methacrylate, isopentyl methacrylate, methyl 3,3-dimethylacrylate, and isopropenyl methyl ketone from the viewpoint of quality stability of methyl methacrylate-containing composition during storage.

In embodiments of the fourth aspect, one or two or more component(s) B3 may be contained in the methyl methacrylate-containing composition.

In cases where the methyl methacrylate-containing composition comprises a compound that corresponds to both components A1 and B3, the compound is considered as the component A1. Thus, the methyl methacrylate-containing composition must comprise a component B3 other than the compound. In cases where two or more compounds that correspond to both components A1 and B3 are contained, the compound with the highest molar concentration in the methyl methacrylate-containing composition is considered as the component A1, and the other compound(s) is/are considered as the component(s) B3.

(Concentrations of Component A1 and Component B3)

When the concentration of the component B3 is MB3 (umol/L), the MB3/MA1 is preferably 0.01 or more, more preferably 0.1 or more, and still more preferably 0.5 or more from the viewpoint of efficiency in reducing the generation of methyl pyruvate. The upper limit of the MB3/MA1 is preferably 1,000 or less, more preferably 500 or less, still more preferably 100 or less, particularly preferably 50 or less, and most preferably 10 or less.

In preferred aspects, the MA1 is the same as the first aspect.

The MB3 is preferably from 1 to 85,000 μmol/L. When the MB3 is 1 μmol/L or more, the effect of reducing the generation of methyl methacrylate dimer and methyl pyruvate can be sufficiently obtained. When the MB3 is 85,000 μmol/L or less, the impurity content after the production of methyl methacrylate polymer by polymerization of the methyl methacrylate-containing composition according to the present embodiment is reduced, so that adverse effects on physical properties of the polymer can be prevented. The lower limit of the MB3 is more preferably 10 μmol/L or more, and still more preferably 50 μmol/L or more. The upper limit of the MB3 is more preferably 40,000 μmol/L or less, still more preferably 20,000 μmol/L or less, and particularly preferably 15,000 μmol/L or less.

(Concentration of Methyl Methacrylate)

In preferred aspects, the concentration of methyl methacrylate is the same as the first aspect.

(Component C)

In preferred aspects, the component C is the same as the first aspect.

(Analysis of Methyl Methacrylate-containing Composition)

The method of confirming that the methyl methacrylate-containing composition contains a component A1, a component B3, a component C, and water, and the method of measuring the concentrations of methyl methacrylate, a component A1, a component B3, a component C, and water are the same as the first aspect.

[Method of Producing Methyl Methacrylate-containing Composition]

The method of producing the methyl methacrylate-containing composition according to the present embodiment may be a method in which a component A1 or a component A2 (hereinafter correctively referred to as "component A"), a component B1, a component B2 or a component B3 (hereinafter correctively referred to as "component B") are added to methyl methacrylate. Methyl methacrylate to be used may be a commercially available product, or methyl methacrylate produced by known methods such as the acetone cyanohydrin (ACH) method, the new acetone cyanohydrin (new ACH) method, the C4 direct oxidation method, the direct methyl esterification method, the ethylene method, and the new ethylene method may be used. The component A and component B to be used may be commercially available products, or those synthesized by known methods may be used. When methyl methacrylate is produced by a known method such as the acetone cyanohydrin (ACH) method, the new acetone cyanohydrin (new ACH) method, the C4 direct oxidation method, the direct methyl esterification method, the ethylene method, or the new ethylene method is used, the component A or the component B may be added raw materials or in the course of the production process to produce the methyl

methacrylate-containing composition. In cases where a component A or a component B is generated as a by-product during the methyl methacrylate production process, a part of the component A or the component B generated may be left for the production of the methyl methacrylate-containing composition.

[Methods of Evaluating Storage Stability and Heat Stability]

The methyl methacrylate-containing composition according to the present embodiment has high quality stability during storage. The method of evaluating the quality stability of the methyl methacrylate-containing composition during storage may be, for example, a method in which the methyl methacrylate-containing composition is actually stored for a long period, and the amounts of methyl methacrylate dimers and methyl pyruvate generated are determined. From the viewpoint of work simplicity, a method may be used in which the methyl methacrylate-containing composition is heated for a short time, and the amounts of methyl methacrylate dimers and methyl pyruvate generated are determined. In cases of heating for short time, the heating temperature is preferably from 50 to 100°C, and the heating time period is preferably from 1 to 24 hours. In the present invention, the quality stability of the methyl methacrylate-containing composition during storage is evaluated based on the amounts of methyl methacrylate dimers and methyl pyruvate generated when the methyl methacrylate-containing composition is stored at 25°C for 14 days.

[Method of Storing Methyl Methacrylate-containing Composition]

The method of storing the methyl methacrylate-containing composition according to the present embodiment comprises storing the methyl methacrylate-containing composition according to the present embodiment at 0 to 50°C. Preferably, the lower limit of the storage temperature is 5°C or more. The upper limit of the storage temperature is preferably 45°C or less, and more preferably 40°C or less.

The storage time period is not particularly limited, and the method is suitable, for example, in cases where the methyl methacrylate-containing composition is stored for 1 day or longer. The methyl methacrylate-containing composition may be stored for 7 days or longer, or for 30 days or longer. The upper limit of the storage time period is not particularly limited, and is, for example, preferably 3 years or shorter, more preferably 1 year or shorter, and still more preferably 90 days or shorter.

[Method of Producing Methyl Methacrylate Polymer]

The method of producing methyl methacrylate polymer according to the present embodiment comprises a step of polymerizing a polymeric composition comprising the methyl methacrylate-containing composition according to the present embodiment.

<Polymeric Composition>

The polymeric composition may comprise, as needed, a monomer that can be copolymerized with methyl methacrylate, and other additives.

(Monomer that can be Copolymerized with Methyl Methacrylate)

Examples of the monomer that can be copolymerized with methyl methacrylate include the following:

a methacrylate ester, such as ethyl methacrylate, isopropyl methacrylate, n-butyl methacrylate, iso-butyl methacrylate, tert-butyl methacrylate, 2-ethylhexyl methacrylate, phenyl methacrylate, or benzyl methacrylate;
an acrylate ester, such as methyl acrylate, ethyl acrylate, n-butyl acrylate, iso-butyl acrylate, tert-butyl acrylate, or 2-ethylhexyl acrylate;
an unsaturated carboxylic acid, such as acrylic acid, methacrylic acid, maleic acid, or itaconic acid;
an unsaturated carboxylic anhydride, such as maleic anhydride, or itaconic anhydride;
maleimide, such as N-phenylmaleimide, or N-cyclohexylmaleimide;
a vinyl monomer containing a hydroxy group, such as 2-hydroxyethyl acrylate, 2-hydroxyethyl methacrylate, or 2-hydroxypropyl methacrylate;
a vinyl ester, such as vinyl acetate, or vinyl benzoate;
vinyl chloride, vinylidene chloride, and derivatives thereof;
a vinyl monomer containing nitrogen, such as methacrylamide, or acrylonitrile;
a monomer containing an epoxy group, such as glycidyl acrylate, or glycidyl methacrylate;
an aromatic vinyl monomer, such as styrene, or $\alpha$-methylstyrene;

alkane diol di(meth)acrylate, such as ethylene glycol di(meth)acrylate, 1,2-propylene glycol di(meth)acrylate, 1,3-butyleneglycol di(meth)acrylate, or 1,6-hexanediol di(meth)acrylate;

polyoxyalkylene glycol di(meth)acrylate, such as diethylene glycol di(meth)acrylate, dipropylene glycol di(meth)acrylate, triethylene glycol (meth)acrylate, tetraethylene glycol di(meth)acrylate, polyethylene glycol di(meth)acrylate, or neopentyl glycol di(meth)acrylate;

a vinyl monomer having two or more ethylenic unsaturated bonds in its molecule, such as divinylbenzene;

an unsaturated polyester prepolymer obtained from at least one polycarboxylic acid including ethylenic unsaturated polycarboxylic acid and at least one diol; and

a vinyl ester prepolymer obtained by acrylic modification of an end of an epoxy group.

Among those described above, the monomer that can be copolymerized with methyl methacrylate is preferably at least one selected from the group consisting of the methacrylate esters and the acrylate esters. This enables polymerization of the polymeric composition to obtain a methyl methacrylate polymer with excellent balance of transparency, heat resistance, and moldability. The monomer that can be copolymerized with methyl methacrylate is more preferably an acrylate ester, and particularly preferably at least one selected from the group consisting of methyl acrylate, ethyl acrylate, and n-butyl acrylate.

One or two or more monomer(s) that can be copolymerized with methyl methacrylate may be used. When the component A or the component B is a monomer that can be copolymerized with methyl methacrylate, the component A or the component B may be used as a monomer that can be copolymerized with methyl methacrylate, or a monomer that can be copolymerized with methyl methacrylate apart from the component A or the component B may be used.

The lower limit of the amount of the monomer that can be copolymerized with methyl methacrylate contained in the polymeric composition is preferably 0.01 parts by mass or more with respect to 100 parts by mass of methyl methacrylate. This enables obtaining a methyl methacrylate polymer with high transparency. The upper limit of the amount of the monomer that can be copolymerized with methyl methacrylate with respect to 100 parts by mass of methyl methacrylate is preferably 50 parts by mass or less, more preferably 40 parts by mass or less, and still more preferably 30 parts by mass or less. The lower limit of the amount of the monomer that can be copolymerized with methyl methacrylate with respect to 100 parts by mass of methyl methacrylate is more preferably 0.1 parts by mass or more, and still more preferably 1 part by mass or more.

(Other Additives)

Other additives preferably include a polymerization initiator. Other additives may also include, for example, a chain transfer agent, a mold release agent, a lubricant, a plasticizer, an antioxidant, an antistatic agent, a light stabilizer, an ultraviolet absorber, a flame retardant, a flame retardant promoter, a polymerization inhibitor, a filler, a pigment, a dye, a silane coupling agent, a leveling agent, an antifoam, and a fluorescent agent, as needed. One or two or more other additive(s) may be contained.

Examples of the polymerization initiator include the following:

an azo compound, such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2-2'-azobis(2-methylpropane), 1,1-azobis(cyclohexanecarbonitrile), and dimethyl-2,2'-azobisisobutyrate;

an organic peroxide, such as benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butyl peroxy-2-ethylhexanoate, t-butyl peroxyisobutyrate, t-butyl peroxybenzoate, t-hexyl peroxybenzoate, t-butyl peroxyisopropyl monocarbonate, t-butyl peroxy-3,5,5-trimethyl hexanoate, t-butyl peroxylaurate, t-butyl peroxyacetate, t-hexyl peroxyisopropyl monocarbonate, t-hexyl peroxy-2-ethyl hexanoate, t-amyl peroxy-2-ethyl hexanoate, 1,1,3,3-tetramethyl butyl peroxyethyl hexanoate, 1,1,2-trimethyl propyl peroxy-2-ethyl hexanoate, 1,1,3,3-tetramethyl butyl peroxyisopropyl monocarbonate, 1,1,2-trimethyl propyl peroxyisopropyl monocarbonate, 1,1,3,3-tetramethyl butyl peroxyisononanoate, 1,1,2-trimethyl propyl peroxyisononanoate, di-t-butyl peroxide, di-t-hexyl peroxide, lauroyl peroxide, and dilauroyl peroxide;

a persulfate compound, such as potassium persulfate; and

a redox polymerization initiator.

Among those described above, the polymerization initiator is preferably at least one selected from the group consisting of the azo compound and the organic peroxide from the viewpoints of storage stability, and reactivity with methyl methacrylate and copolymerizable monomers.

The amount of the polymerization initiator used is preferably from 0.0001 to 1 part by mass with respect to 100 parts by mass of the total of methyl methacrylate and the monomer that can be copolymerized with methyl methacrylate.

<Method of Polymerizing Polymeric Composition>

Examples of the polymerization method include a bulk polymerization method, a solution polymerization method, an emulsion polymerization method, and a suspension polymerization method. From the viewpoint of environmental load due to the use of solvents and the like, and of transparency of the methyl methacrylate polymer to be obtained, a bulk polymerization method is preferable.

Specific means for the bulk polymerization method is not particularly limited, and a known casting polymerization method such as a cell casting method or a continuous casting method can be used for production.

The casting polymerization method is a method for obtaining methyl methacrylate polymer by injecting a polymeric composition into a mold composed of two inorganic glass plates or metal plates (for example, SUS plates) placed opposite each other at a predetermined distance with the periphery sealed with a gasket such as a soft resin tube, and allowing the composition to polymerize.

The mold for casting polymerization is not particularly limited, and known molds can be used. Examples of the mold for cell casting include those in which two plate-like bodies, such as inorganic glass plates, chromium-plated metal plates, and stainless steel plates, are placed opposite each other at a predetermined distance, and a gasket is placed around the periphery to allow the plate-like bodies and the gasket to form a sealed space. Examples of the mold for continuous casting include those in which a sealed space is formed by opposing faces of a pair of endless belts running in the same direction at the same speed, and gaskets running at the same speed as the endless belt on both sides of the endless belt.

The spacing of the void in a mold is adjusted as appropriate to obtain a resin sheet with a desired thickness, and is generally from 1 to 30 mm.

The polymerization temperature is preferably from 70 to 210°C. This enables achieving an appropriate polymerization rate. The lower limit of the polymerization temperature is more preferably 80°C or more, still more preferably 100°C or more, particularly preferably 125°C or more, and most preferably 130°C or more. The upper limit is more preferably 180°C or less, and still more preferably 150°C or less. The polymerization time is not particularly limited, and can be, for example, from 0.5 to 24 hours.

EXAMPLES

The present invention will be described in detail with reference to Examples and Comparative Examples below, but the present invention is not limited to these Examples. Unless otherwise stated, the terms "%" and "ppm" in the examples and the comparative examples mean "% by weight" and "ppm by weight," respectively.

The water concentration contained in a methyl methacrylate reagent was determined by the Karl-Fischer method. The composition of the components of the methyl methacrylate-containing composition before being stored was determined based on the amounts of the raw materials added. Methyl methacrylate dimers and methyl pyruvate in the methyl methacrylate-containing composition after being stored were determined by an absolute calibration curve method using GC-MS. The measurement conditions for GC-MS are shown below.

Instrument: GC-MS measuring system (product name: QP-2010SE, manufactured by Shimadzu Corporation)

[Conditions for GC]

Column (product name: DB-WAX, manufactured by Agilent Technologies, Inc.);
Length: 60 m, Inner diameter: 0.32 mm, Film thickness: 1.00 um;
Injection volume: 1.0 μL;
Vaporizing chamber temperature: 210°C;
Column oven temperature: held at 35°C for 10 minutes, raised from 35°C to 150°C at 5°C/min, held at 150°C for 17 minutes, raised from 150°C to 220°C at 5°C/min, and held at 220°C for 6 minutes;
Carrier gas: helium;
Injection mode: split (split ratio: 50);
Control mode: constant linear velocity (25.0 cm/sec);
Pressure: 26.1 KPa;
Total flow: 52.5 mL/min;
Purge flow: 3.0 mL/min; and
Column flow: 0.97 mL/min.

[Conditions for MS]

Ionization method: EI (Electron Ionization);
Ion source temperature: 250°C;
Interface temperature: 250°C;
m/z detection range: 10 to 300; and
Detection time: 70 minutes.

Determination of the water concentration by the Karl-Fischer method was carried out using automated water measurement equipment (product name: AQV-2200, manufactured by HIRANUMA Co., Ltd.).

(Example 1)

Using 2,3,5,6-tetramethylpyrazine as a component A, 0.0214 g of the component A was added to 10.0196 g of a methyl methacrylate reagent (water concentration: 240 ppm) to prepare a methyl methacrylate solution (A-1 solution). The concentration of the component A in the A-1 solution is shown in Table 1.

Using 2,4-dimethyl-6-t-butylphenol as a component B, 0.0829 g of the component B was added to 40.0221 g of a methyl methacrylate reagent (water concentration: 240 ppm) to prepare a methyl methacrylate solution (B-1 solution). The concentration of the component B in the B-1 solution is shown in Table 1.

Next, 0.1017 g of the A-1 solution and 0.1016 g of the B-1 solution were added to 20.0861 g of a methyl methacrylate reagent (water concentration: 240 ppm) to prepare a methyl methacrylate-containing composition. The concentrations of the components in the methyl methacrylate-containing composition are shown in Table 3. In Table 3, MA1 and MA2 are correctively referred to as "MA", and MB1, MB2, and MB3 are correctively referred to as "MB."

The obtained methyl methacrylate-containing composition was stored at 25°C for 14 days. The amounts of methyl methacrylate dimers and methyl pyruvate generated in the methyl methacrylate-containing composition after the storage are shown in Table 3.

(Examples 2 to 6)

A-1 solutions were prepared in the same manner as in Example 1 except that a compound shown in Table 1 was used as a component A, and the amounts of the methyl methacrylate reagent and the component A were changed as shown in Table 1.

B-1 solutions were prepared in the same manner as in Example 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing compositions after the storage are shown in Table 3.

(Examples 7 to 9)

A-1 solutions were prepared in the same manner as in Example 1 except that a compound shown in Table 1 was used as a component A, and the amounts of the methyl methacrylate reagent and the component A were changed as shown in Table 1.

B-1 solutions were prepared in the same manner as in Example 1 except that the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing composition after the storage are shown in Table 3.

(Examples 10 and 17)

A-1 solutions were prepared in the same manner as in Example 1.

B-1 solutions were prepared in the same manner as in Example 1 except that a compound shown in Table 1 was used as a component B, and the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing compositions after the storage are shown in Table 3.

(Example 18)

A B-1 solution was prepared in the same manner as in Example 1.

Next, using 2,3,5,6-tetramethylpyrazine as a component A, 0.0210 g of the component A and 0.1021 g of the B-1 solution were added to 20.0176 g of a methyl methacrylate reagent (water concentration: 240 ppm) to prepare a methyl methacrylate-containing composition. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Example 19)

A B-1 solution was prepared in the same manner as in Example 1.

Next, a methyl methacrylate-containing composition was prepared in the same manner as in Example 18 except that the amounts of the methyl methacrylate reagent, the component A, and the B-1 solution were changed as shown in Table 2. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Example 20)

An A-1 solution and a B-1 solution were prepared in the same manner as in Example 1.

Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Examples 21 and 22)

A-1 solutions were prepared in the same manner as in Example 1.

B-1 solutions were prepared in the same manner as in Example 1 except that the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing compositions after the storage are shown in Table 3.

(Example 23)

An A-1 solution was prepared in the same manner as in Example 1.

Next, 0.1025 g of the A-1 solution was added to 20.0016 g of a methyl methacrylate reagent (water concentration: 240 ppm) to prepare a methyl methacrylate-containing composition. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Example 24)

An A-1 solution was prepared in the same manner as in Example 1 except that a compound shown in Table 1 was used as a component A, and the amounts of the methyl methacrylate reagent and the component A were changed as shown in Table 1.

A B-1 solution was prepared in the same manner as in Example 1 except that the amounts of the methyl methacrylate reagent and the component B were changed as shown in Table 1.

Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Examples 25 and 26)

A-1 solutions were prepared in the same manner as in Example 1 except that a compound shown in Table 1 was used as a component A, and the amounts of the methyl methacrylate reagent and the component A were changed as shown in Table 1.

Next, methyl methacrylate-containing compositions were prepared in the same manner as in Example 23 except that the amounts of the methyl methacrylate reagent and the A-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing compositions after the storage are shown in Table 3.

(Examples 27 to 29)

A-1 solutions were prepared in the same manner as in Example 1.

B-1 solutions were prepared in the same manner as in Example 1 except that a compound shown in Table 1 was used as a component B, and the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing compositions after the storage are shown in Table 3.

(Examples 30 to 33)

A-1 solutions were prepared in the same manner as in Example 1 except that a compound shown in Table 1 was used as a component A, and the amounts of the methyl methacrylate reagent and the component A were changed as shown in Table 1.

B-1 solutions were prepared in the same manner as in Example 1 except that a compound shown in Table 1 was used as a component B, and the amounts of the methyl methacrylate reagent and the component B were changed as shown in Table 1.

Next, methyl methacrylate-containing compositions were prepared in the same manner as in Example 1 except

that the amounts of the methyl methacrylate reagent, the A-1 solution, and the B-1 solution were changed as shown in Table 2. The concentrations of the components in the methyl methacrylate-containing compositions are shown in Table 3.

The obtained methyl methacrylate-containing compositions were stored as in Example 1. The amounts of methyl methacrylate dimers and methyl pyruvate generated in the methyl methacrylate-containing compositions after the storage are shown in Table 3.

(Comparative Example 1)

A B-1 solution was prepared in the same manner as in Example 1 except that the amounts of the methyl methacrylate reagent and the component B were changed as shown in Table 1.

Next, 0.2213 g of the B-1 solution was added to 40.0273 g of a methyl methacrylate reagent (water concentration: 240 ppm) to prepare a methyl methacrylate-containing composition. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Comparative Example 2)

40.0000 g of a methyl methacrylate reagent (water concentration: 240 ppm) was used as a methyl methacrylate-containing composition and stored as in Example 1. The amounts of methyl methacrylate dimers and methyl pyruvate generated in the methyl methacrylate-containing composition after the storage are shown in Table 3.

(Comparative Example 3)

A B-1 solution was prepared in the same manner as in Comparative Example 1.

Using diacetyl as a component C, 0.0205 g of the component C was added to 9.9913 g of a methyl methacrylate reagent (water concentration: 240 ppm) to prepare a methyl methacrylate solution (C-1 solution). The concentration of the component C in the C-1 solution is shown in Table 1.

Next, 0.2151 g of the B-1 solution and 0.2069 g of the C-1 solution were added to 40.0218 g of a methyl methacrylate reagent (water concentration: 240 ppm) to prepare a methyl methacrylate-containing composition. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Comparative Example 4)

A B-1 solution was prepared in the same manner as in Example 1.

Next, a methyl methacrylate-containing composition was prepared in the same manner as in Example 18 except that the amounts of the methyl methacrylate reagent, the component A, and the B-1 solution were changed as shown in Table 2. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Comparative Example 5)

An A-1 solution and a B-1 solution were prepared in the same manner as in Example 1.

Next, 0.0980 g of the A-1 solution, 0.1014 g of the B-1 solution, and 0.2940 g of pure water were added to 20.0017 g of a methyl methacrylate reagent (water concentration: 240 ppm) to prepare a methyl methacrylate-containing composition. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

(Comparative Example 6)

An A-1 solution was prepared in the same manner as in Example 24.

Next, a methyl methacrylate-containing composition was prepared in the same manner as in Example 23 except that the amounts of the methyl methacrylate reagent and the A-1 solution were changed as shown in Table 2. The concentrations of the components in the methyl methacrylate-containing composition are shown in Table 3.

The obtained methyl methacrylate-containing composition was stored as in Example 1. The amounts of methyl methacrylate dimers and methyl pyruvate generated in the methyl methacrylate-containing composition after the storage are shown in Table 3.

Table 1

| | A-1 solution | | | | B-1 solution | | | | C-1 solution | | | |
|---|---|---|---|---|---|---|---|---|---|---|---|---|
| | Amount of MMA regent added [g] | Component A | | | Amount of MMA regent added [g] | Component B | | | Amount of MMA regent added [g] | Component C | | |
| | | Compound name | Added amount [g] | Concentration [ppm] | | Compound name | Added amount [g] | Concentration [ppm] | | Compound name | Added amount [g] | I Concentration [ppm] |
| Example 1 | 10.0196 | 2,3,5,6-Tetramethylpyradine | 0.0214 | 2131 | 40.0221 | 2,4-Dimethyl-6-t-butylphenol | 0.0829 | 2067 | - | - | - | - |
| Example 2 | 10.0077 | Pyradine | 0.0240 | 2392 | 40.0221 | 2,4-Dimethyl-6-t-butylphenol | 0.0829 | 2067 | - | - | - | - |
| Example 3 | 10.0077 | 2,3,5-Trimethylpyradine | 0.0224 | 2233 | 40.0221 | 2,4-Dimethyl-6-t-butylphen $_0$1 | 0.0829 | 2067 | - | - | - | - |
| Example 4 | 10.0294 | 2-Methoxypyrazine | 0.0205 | 2040 | 40.0221 | 2.4-Di methyl-6-t-butylphenol | 0.0829 | 2067 | - | - | - | - |
| Example 5 | 10.0278 | 2-Isopropyl-3-methoxypyrazine | 0.0210 | 2090 | 40.0221 | 2,4-Dimethyl-6-t-butylphenol | 0.0829 | 2067 | - | - | - | - |
| Example 6 | 10.0057 | 2,5-Dimethylpyradine | 0.0210 | 2094 | 40.0221 | 2,4-Dimethyl-6-t-butylphenol | 0.0829 | 2067 | - | - | - | - |
| Example 7 | 10.0155 | 2-Aminopyrazine | 0.0201 | 2003 | 40.0080 | 2,4-Dimethyl-6-t-butylphenol | 0.0828 | 2065 | - | - | - | - |
| Example 8 | 10.0062 | 2-(Methylthio)pyrazine | 0.0236 | 2353 | 40.0080 | 2,4-Dimethyl-6-t-butylphenol | 0.0828 | 2065 | - | - | - | - |
| Example 9 | 10.0116 | 2-Pyrazinemethanol | 0.0210 | 2093 | 40.0080 | 2,4-Di methyl-6-t-butylphenol | 0.0828 | 2065 | - | - | - | - |
| Example 10 | 10.0196 | 2,3,5,6-Tetramethylpyradine | 0.0214 | 2131 | 10.0051 | 4-Methoxyphenol | 0.0191 | 1905 | - | - | - | - |
| Example 11 | 10.0196 | 2,3,5,6-Tetramethylpyradine | 0.0214 | 2131 | 10.0238 | Hydroquinone | 0.0233 | 2319 | - | - | - | - |
| Example 12 | 10.0196 | 2,3,5,6-Tetramethylpyradine | 0.0214 | 2131 | 10.0204 | Phenothiazine | 0.0238 | 2370 | - | - | - | - |

(continued)

| | A-1 solution | | | | B-1 solution | | | | C-1 solution | | | |
|---|---|---|---|---|---|---|---|---|---|---|---|---|
| | Amountof MMA re-gent add-ed [g] | Component A | | | Amountof MMA re-gent add-ed [g] | Component B | | | Amount of MMA regent added [g] | Component C | | |
| | | Compound name | Added amount [g] | Concent ration [ppm] | | Compound name | Added amount [g] | Concent ration [ppm] | | Compound name | Added amount [g] | I Concent ration [ppm] |
| Example 13 | 10.0196 | 2,3,5,6-Tetramethylpyradine | 0.0214 | 2131 | 10.0021 | 4-Hydroxy-2,2,6,6-tetramethylpiperidine-N-oxyl | 0.0202 | 2016 | - | - | - | - |
| Example 14 | 10.0196 | 2,3,5,6-Tetramethylpyradine | 0.0214 | 2131 | 10.1244 | N,N-Diphenylamine | 0.0222 | 2188 | - | - | - | - |
| Example 15 | 10.0196 | 2,3,5,6-Tetramethylpyradine | 0.0214 | 2131 | 10.0077 | N-Nitrosodiphenylamine | 0.0208 | 2074 | - | - | - | - |
| Example 16 | 10.0196 | 2,3,5,6-Tetramethylpyradine | 0.0214 | 2131 | 10.0061 | Triphenyl phosphite | 0.0223 | 2224 | - | - | - | - |
| Example 17 | 10.0196 | 2,3,5,6-Tetramethylpyradine | 0.0214 | 2131 | 40.0221 | 2,4-Dimethyl-6-t-butylphenol | 0.0829 | 2067 | - | - | - | - |
| Example 18 | | | | | 40.0221 | 2,4-Dimethyl-6-t-butylphenol | 0.0829 | 2067 | - | - | - | - |
| Example 19 | | | | | 40.0221 | 2,4-Dimethyl-6-t-butylphenol | 0.0829 | 2067 | - | - | - | - |
| Example 20 | 10.0196 | 2,3,5,6-Tetramethylpyradine | 0.0214 | 2131 | 40.0221 | 2.4- Di me th yi-6-t-butylphenol | 0.0829 | 2067 | - | - | - | - |
| Example 21 | 10.0196 | 2,3,5,6-Tetramethylpyradine | 0.0214 | 2131 | 40.0080 | 2,4-Dimethyl-6-t-butylphenol | 0.0828 | 2065 | - | - | - | - |
| Example 22 | 10.0196 | 2,3,5,6-Tetramethylpyradine | 0.0214 | 2131 | 40.0080 | 2.4-Dimethyl-6-t-butylphenol | 0.0828 | 2065 | - | - | - | - |
| Example 23 | 10.0196 | 2,3,5,6-Tetramethylpyradine | 0.0214 | 2131 | | | | | - | - | - | - |

Table 1 (cont)

| | A-1 solution | | | | B-1 solution | | | | C-1 solution | | | |
|---|---|---|---|---|---|---|---|---|---|---|---|---|
| | Amount of MMA re-gent add-ed [g] | Component A | | | Amount of MMA re-gent add-ed [g] | Component B | | | Amount of MMA re-gent add-ed [g] | Component C | | |
| | | Compound name | Added amount [g] | Concent ration [ppm] | | Compound name | Added amount [g] | Concent ration [ppm] | | Compound name | Added amount [g] | Concent ration [ppm] |
| Example 24 | 10.0051 | 2-Vinylpyrazine | 0.0206 | 2055 | 40.0080 | 2,4-Dimethyl-6-t-butylphenol | 0.0828 | 2065 | - | - | - | - |
| Example 25 | 10.0077 | 2,3,5-Trimethylpyradine | 0.0224 | 2233 | - | - | - | - | - | - | - | - |
| Example 26 | 10.0278 | 2-Isopropyl-3-methoxypyrazine | 0.0210 | 2090 | - | - | - | - | - | - | - | - |
| Example 27 | 10.0196 | 2,3,5,6-Tetramethylpyradine | 0.0214 | 2131 | 10.0822 | Methyl isobutyrate | 0.0293 | 2898 | - | - | - | - |
| Example 28 | 10.0196 | 2,3,5,6-Tetramethylpyradine | 0.0214 | 2131 | 10.1504 | Methyl propionate | 0.0179 | 1760 | - | - | - | - |
| Example 29 | 10.0196 | 2,3,5,6-Tetramethylpyradine | 0.0214 | 2131 | 10.0701 | Methyl acrylate | 0.0186 | 1844 | - | - | - | - |
| Example 30 | 10.0077 | 2,3,5-Trimethylpyradine | 0.0224 | 2233 | 10.0822 | Methyl isobutyrate | 0.0293 | 2898 | - | - | - | - |
| Example 31 | 10.0077 | 2,3,5-Trimethylpyradine | 0.0224 | 2233 | 10.1504 | Methyl propionate | 0.0179 | 1760 | - | - | - | - |
| Example 32 | 10.0077 | 2,3,5-Trimethylpyradine | 0.0224 | 2233 | 10.0701 | Methyl acrylate | 0.0186 | 1844 | - | - | - | - |
| Example 33 | 10.0077 | 2,3,5-Trimethylpyradine | 0.0224 | 2233 | 10.0335 | Ethyl methacrylate | 0.0197 | 1960 | - | - | - | - |
| Comparative example 1 | - | - | - | - | 10.0026 | 2,4-Dimethyl-6-t-butylphenol | 0.0228 | 2274 | - | - | - | - |
| Comparative example 2 | - | - | - | - | - | - | - | - | -- | - | - | - |
| Comparative example 3 | - | - | - | - | 10.0026 | 2.4-Dimethyl-6-t-butylphenol | 0.0228 | 2274 | 9.9913 | Diacetyl | 0.0205 | 2048 |

(continued)

| | A-1 solution | | | | B-1 solution | | | | C-1 solution | | | |
|---|---|---|---|---|---|---|---|---|---|---|---|---|
| | Amount of MMA reagent added [g] | Component A | | | Amount of MMA reagent added [g] | Component B | | | Amount of MMA reagent added [g] | Component C | | |
| | | Compound name | Added amount [g] | Concentration [ppm] | | Compound name | Added amount [g] | Concentration [ppm] | | Compound name | Added amount [g] | Concentration [ppm] |
| Comparative example 4 | - | - | - | - | 40.0221 | 2,4-Dimethyl-6-t-butylphenol | 0.0829 | 2067 | - | - | - | - |
| Comparative example 5 | 10.0196 | 2,3,5,6-Tetramethylpyradine | 0.0214 | 2131 | 40.0221 | 2,4-Dimethyl-6-t-butylphenol | 0.0829 | 2067 | - | - | - | - |
| Comparative example 6 | 10.0051 | 2-Vinylpyrazine | 0.0206 | 2055 | - | - | - | - | - | - | - | - |

Table 2

| | Added amount [g] | | | | | |
|---|---|---|---|---|---|---|
| | MMA reagent | A-1 solution | 8-1 solution | C-1 solution | Compo nent A | Pure water |
| Example 1 | 20.0861 | 0.1017 | 0.1016 | - | - | - |
| Example 2 | 20.0261 | 0.1039 | 0.1071 | - | - | - |
| Example 3 | 20.0129 | 0.1014 | 0.1045 | - | - | - |
| Example 4 | 20.1391 | 0.1038 | 0.1071 | - | - | - |
| Example 5 | 20.0117 | 0.1059 | 0.1012 | - | - | - |
| Example 6 | 20.0510 | 0.1067 | 0.1038 | - | - | - |
| Example 7 | 10.0111 | 0.0530 | 0.0509 | - | - | - |
| Example 8 | 10.0070 | 0.0523 | 0.0536 | - | - | - |
| Example 9 | 10.0038 | 0.0521 | 0.0516 | - | - | - |
| Example 10 | 20.0510 | 0.1014 | 0.1056 | - | - | - |
| Example 11 | 20.0161 | 0.1013 | 0.1023 | - | - | - |
| Example 12 | 20.0180 | 0.1032 | 0.1051 | - | - | - |
| Example 13 | 10.0020 | 0.0532 | 0.0520 | - | - | - |
| Example 14 | 10.0271 | 0.0513 | 0.0523 | - | - | - |
| Example 15 | 10.0154 | 0.0553 | 0.0521 | - | - | - |
| Example 16 | 10.0055 | 0.0534 | 0.0508 | - | - | - |
| Example 17 | 19.0407 | 1.0019 | 0.1055 | - | - | - |
| Example 18 | 20.0176 | - | 0.1021 | - | 0.0210 | - |
| Example 19 | 9.9970 | - | 0.0546 | - | 0.0926 | - |
| Example 20 | 19.0146 | 0.1034 | 1.0058 | - | - | - |
| Example 21 | 7.5067 | 0.0551 | 2.5107 | - | - | - |
| Example 22 | 4.0066 | 0.0605 | 5.9942 | - | - | - |
| Example 23 | 20.0016 | 0.1025 | - | - | - | - |
| Example 24 | 20.0111 | 0.1008 | 0.1050 | - | - | - |
| Example 25 | 20.0240 | 0.1018 | - | - | - | - |
| Example 26 | 20.0203 | 0.1046 | - | - | - | - |
| Example 27 | 20.0293 | 0.1030 | 0.0722 | - | - | - |
| Example 28 | 20.0626 | 0.1036 | 0.1028 | - | - | - |
| Example 29 | 20.0185 | 0.1068 | 0.1019 | - | | |
| Example 30 | 20.0180 | 0.1029 | 0.0713 | - | - | - |
| Example 31 | 20.0045 | 0.1009 | 0.1058 | - | - | - |
| Example 32 | 20.0265 | 0.1018 | 0.1010 | - | - | - |
| Example 33 | 20.1004 | 0.1035 | 0.1011 | - | - | - |
| Comparative example 1 | 40.0273 | - | 0.2213 | - | - | - |
| Comparative example 2 | 40.0000 | - | - | - | - | - |

(continued)

| | Added amount [g] | | | | | |
|---|---|---|---|---|---|---|
| | MMA reagent | A-1 solution | 8-1 solution | C-1 solution | Compo nent A | Pure water |
| Comparative example 3 | 40.0218 | - | 0.2151 | 0.2069 | - | - |
| Comparative example 4 | 20.0342 | - | 0.1012 | - | 0.3141 | - |
| Comparative example 5 | 20.0017 | 0.0980 | 0.1014 | - | - | 0.2940 |
| Comparative example 6 | 20.0184 | 0.1018 | - | - | - | - |

Table 3

| | MMA-containing composition | | | | | | | | Amount of MMA dimer generated [ppm] | Amount of methyl pyruvate generated [ppm] |
|---|---|---|---|---|---|---|---|---|---|---|
| | MMA | Component A | | Component B | | Component C | | MB/MA | | |
| | Concentration [%] | Compound name | Concentration MA [μmol/L] | Compound name | Concentration MB [μmol/L] | Compound name | Concentration [μmol/L] | | | |
| Example 1 | 99.97 | 2,3,5,6-Tetramethylpyradine | 74 | 2,4-Dimethyl-6-t-butylphenol | 55 | - | - | 0.740 | 18 | 0 |
| Example 2 | 99.97 | Pyradine | 145 | 2,4-Dimethyl-6-t-butylphenol | 58 | - | - | 0.400 | 16 | 1 |
| Example 3 | 99.97 | 2,3,5-Trimethylpyradine | 87 | 2,4-Dimethyl-6-t-butylphenol | 57 | - | - | 0.654 | 12 | 0 |
| Example 4 | 99.97 | 2-Methoxypyrazine | 89 | 2,4-Dimethyl-6-t-butylphenol | 58 | - | - | 0.646 | 15 | 0 |
| Example 5 | 99.97 | 2-Isopropyl-3-methoxypyrazine | 68 | 2,4-Dimethyl-6-t-butylphenol | 55 | - | - | 0.807 | 19 | 1 |
| Example 6 | 99.97 | 2,5-Dimethylpyradine | 96 | 2,4-Dimethyl-6-t-butylphenol | 56 | - | - | 0.582 | 17 | 2 |
| Example 7 | 99.97 | 2-Aminopyrazine | 104 | 2,4-Dimethyl-6-t-butylphenol | 55 | - | - | 0.528 | 25 | 10 |
| Example 8 | 99.97 | 2-(Methylthio) pyrazine | 91 | 2,4-Dimethyl-6-t-butylphenol | 58 | - | - | 0.637 | 21 | 2 |
| Example 9 | 99.97 | 2-Pyrazinemethanol | 92 | 2,4-Dimethyl-6-t-butylphenol | 56 | - | - | 0.604 | 21 | 5 |
| Example 10 | 99.97 | 2,3,5,6-Tetramethylpyradine | 74 | 4-Methoxyphenol | 76 | - | - | 1.021 | 14 | 1 |
| Example 11 | 99.97 | 2,3,5,6-Tetramethylpyradine | 74 | Hydroquinone | 101 | - | - | 1.359 | 17 | 1 |
| Example 12 | 99.97 | 2,3,5,6-Tetramethylpyradine | 75 | Phenothiazine | 58 | - | - | 0.774 | 19 | 1 |

(continued)

| | MMA-containing composition | | | | | | | | Amount of MMA dimer generated [ppm] | Amount of methyl pyruvate generated [ppm] |
|---|---|---|---|---|---|---|---|---|---|---|
| | MMA | Component A | | Component B | | Component C | | MB/MA | | |
| | Concentration [%] | Compound name | Concentration MA [μmol/L] | Compound name | Concentration MB [μmol/L] | Compound name | Concentration [μmol/L] | | | |
| Example 13 | 99.97 | 2, 3, 5,6-Tetramethylpyradine | 78 | 4-Hydroxy-2,2,6,6-tetramethyl piperidine-N-oxyl | 57 | - | - | 0.731 | 15 | 4 |
| Example 14 | 99.97 | 2,3,5,6-Tetramethylpyradine | 75 | N,N-Diphenylamine | 63 | - | - | 0.842 | 0 | 0 |
| Example 15 | 99.97 | 2,3,5,6-Tetramethylpyradine | 81 | N-Nitrosodiphenylamine | 51 | - | - | 0.630 | 20 | 5 |
| Example 16 | 99.97 | 2,3,5,6-Tetramethylpyradine | 78 | Triphenyl phosphite | 34 | - | - | 0.436 | 20 | 9 |
| Example 17 | 99.96 | 2,3,5,6-Tetramethylpyradine | 735 | 2,4-Dimethyl-5-t-butylphenol | 57 | - | - | 0.078 | 19 | 1 |
| Example 18 | 99.87 | 2,3,5,6-Tetramethylpyradine | 7227 | 2,4-Dimethyl-6-t-butylphenol | 55 | - | - | 0.008 | 16 | 1 |
| Example 19 | 99.06 | 2,3,5,6-Tetramethylpyradine | 63269 | 2,4-Dimethyl-6-t-butylphenol | 59 | - | - | 0.001 | 21 | 3 |
| Example 20 | 99.96 | 2,3,5,6-Tetramethylpyradine | 76 | 2,4-Dimethyl-6-t-butylphenol | 547 | - | - | 7.208 | 20 | 0 |
| Example 21 | 99.92 | 2,3,5,6-Tetramethylpyradine | 81 | 2,4-Dimethyl-6-t-butylphenol | 2726 | - | - | 33.734 | 25 | 0 |
| Example 22 | 99.85 | 2,3,5,6-Tetramethylpyradine | 89 | 2,4-Dimethyl-6-t-butylphenol | 6515 | - | - | 73.350 | 26 | 0 |
| Example 23 | 99.97 | 2,3,5,6-Tetramethylpyradine | 75 | - | - | - | - | - | 18 | 4 |

Table 3 (cont.)

| | MMA-containing composition | | | | | | | | Amount of MMA dimer generated [ppm] | Amount of methyl pyruvate generated [ppm] |
|---|---|---|---|---|---|---|---|---|---|---|
| | MMA | Component A | | Component B | | Component C | | MB/MA | | |
| | Concent ration [%] | Compound name | Concent ration MA [μmol/L] | Compound name | Concent ration MB [μmol/L] | Compound name | Concent ration [μmol/L] | | | |
| Example 24 | 99.97 | 2-Vinylpyrazine | 91 | 2,4-Dimethyl-6-t-butylphenol | 57 | - | - | 0.623 | 14 | 3 |
| Example 25 | 99.97 | 2,3,5-Trimethylpyradine | 87 | - | - | - | - | - | 17 | 12 |
| Example 26 | 99.97 | 2-Isopropyl-3-methoxypyrazine | 67 | - | - | - | - | - | 17 | 11 |
| Example 27 | 99.97 | 2,3, 5,6-Tetramethylpyradine | 75 | Methyl isobutyrate | 96 | - | - | 1.271 | 18 | 9 |
| Example 28 | 99.97 | 2,3,5,6-Tetramethylpyradine | 76 | Methyl propionate | 96 | - | - | 1.267 | 20 | 15 |
| Example 29 | 99.97 | 2,3,5,6-Tetramethylpyradine | 78 | Methyl acrylate | 102 | - | - | 1.306 | 21 | 13 |
| Example 30 | 99.97 | 2,3,5-Trimethylpyradine | 88 | Methyl isobutyrate | 95 | - | - | 1.075 | 19 | 16 |
| Example 31 | 99.97 | 2,3,5-Trimethylpyradine | 86 | Methyl propionate | 99 | - | - | 1.146 | 20 | 12 |
| Example 32 | 99.97 | 2,3,5-Trimethylpyradine | 87 | Methyl acrylate | 101 | - | - | 1.162 | 13 | 16 |
| Example 33 | 99.97 | 2,3,5-Trimethylpyradine | 88 | Ethyl methacrylate | 81 | - | - | 0.917 | 21 | 14 |
| Comparative example 1 | 99.97 | - | - | 2,4-Dimethyt-6-t-butylphenol | 66 | - | - | - | 30 | 7 |
| Comparative example 2 | 99.98 | - | - | - | - | - | - | - | 0 | 25 |

(continued)

| | MMA-containing composition | | | | | | | | Amount of MMA dimer generated [ppm] | Amount of methyl pyruvate generated [ppm] |
|---|---|---|---|---|---|---|---|---|---|---|
| | MMA | Component A | | Component B | | Component C | | MB/MA | | |
| | Concentration [%] | Compound name | Concentration MA [μmol/L] | Compound name | Concentration MB [μmol/L] | Compound name | Concentration [μmol/L] | | | |
| Comparative example 3 | 99.97 | - | - | 2,4-Dimethyl-6-t-butylphenol | 64 | Diacetyl | 115 | - | 0 | 1056 |
| Comparative example 4 | 98.44 | 2,3,5,6-Tetramethylpyradine | 106458 | 2,4-Dimethyl-6-t-butylphenol | 54 | - | - | 0.001 | 33 | 11 |
| Comparative example 5 | 98.54 | 2,3, 5,6-Tetramethylpyradine | 71 | 2,4-Dimethyl-6-t-butylphenol | 54 | - | - | 0.767 | 33 | 6 |
| Comparative example 6 | 99.97 | 2-Vinylpyrazine | 92 | - | - | - | - | - | 31 | 30 |

As shown in Tbale 1, 2, and 3, Examples 1 to 33 in which the methyl methacrylate-containing composition contains defined component A and component B show a reduction of both generation of methyl methacrylate dimers and methyl pyruvate in the methyl methacrylate-containing compositions after storage, and can be said to have high quality stability during storage. Examples 23, 25, and 26 containing a defined concentration of a component corresponding to the component A2 show reduction of both generation of methyl methacrylate dimers and methyl pyruvate in the methyl methacrylate-containing composition after storage even without a component B, and can be said to have high quality stability during storage.

The polymeric composition comprising each methyl methacrylate-containing composition obtained in each Example can be polymerized to obtain a methyl methacrylate polymer.

INDUSTRIAL APPLICABILITY

According to the present invention, methyl methacrylate-containing compositions that can be used, for example, as raw materials for acrylic resins can be stored stably for a long period of time, which is industrially useful.

## Claims

1. A methyl methacrylate-containing composition, comprising:

   methyl methacrylate;
   a pyrazine compound represented by the following Formula (1) (component A1); and
   a polymerization inhibitor (component B1),
   wherein the concentration of methyl methacrylate is from 99 to 99.99% by mass,

   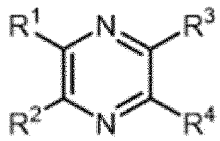
   (1)

   wherein, in Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group.

2. The methyl methacrylate-containing composition according to claim 1, wherein when the concentration of the component A1 is MA1 (umol/L), and the concentration of the component B1 is MB1 (umol/L), the MB1/MA1 is from 0.001 to 100.

3. The methyl methacrylate-containing composition according to claim 1, wherein when the concentration of the component A1 is MA1 (umol/L), the MA1 is from 1 to 65,000 pmol/L.

4. The methyl methacrylate-containing composition according to claim 3, wherein the MA1 is from 10 to 30,000 pmol/L.

5. The methyl methacrylate-containing composition according to claim 1, wherein when the concentration of the component B1 is MB1 (umol/L), the MB1 is from 1 to 7,000 pmol/L.

6. The methyl methacrylate-containing composition according to claim 5, wherein the MB1 is from 10 to 1,000 pmol/L.

7. The methyl methacrylate-containing composition according to claim 1, wherein the molecular weight of the component A1 is 1,000 or less.

8. The methyl methacrylate-containing composition according to claim 1, wherein the component B1 is at least one polymerization inhibitor selected from the group consisting of a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound.

9. The methyl methacrylate-containing composition according to claim 1, wherein the component B1 is at least one

polymerization inhibitor selected from the group consisting of a phenol compound, an N-oxyl compound, an amine compound, a phosphorus-containing compound, and a sulfur-containing compound.

**10.** The methyl methacrylate-containing composition according to claim 1, wherein the component B1 is at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine.

**11.** The methyl methacrylate-containing composition according to claim 1, wherein in the Formula (1), $R^1$, $R^2$, $R^3$, and $R^4$ are each independently a hydrogen atom, a $C_{1-5}$ alkyl group, a $C_{2-5}$ alkenyl group, a $C_{1-12}$ aryl group, a $C_{1-6}$ alkoxy group, a $C_{0-6}$ amino group, a $C_{1-6}$ monovalent group containing a carbonyl group, a $C_{1-5}$ alkylthio group, or a $C_{6-10}$ arylthio group.

**12.** The methyl methacrylate-containing composition according to claim 1, wherein in the Formula (1), $R^1$, $R^2$, $R^3$, and $R^4$ are each independently a hydrogen atom, a $C_{1-5}$ alkyl group, or a $C_{1-6}$ alkoxy group.

**13.** The methyl methacrylate-containing composition according to claim 1, wherein in the Formula (1), $R^1$, $R^2$, $R^3$, and $R^4$ are each independently a hydrogen atom, a methyl group, an ethyl group, an n-propyl group, an isopropyl group, or a methoxy group.

**14.** The methyl methacrylate-containing composition according to claim 1, wherein the concentration of methyl methacrylate is from 99.8 to 99.99% by mass.

**15.** The methyl methacrylate-containing composition according to claim 1, wherein the composition does not contain diacetyl, or the concentration of diacetyl contained is 55 pmol/L or less.

**16.** A methyl methacrylate-containing composition, comprising:

methyl methacrylate; and
a pyrazine compound represented by the following Formula (2) (component A2),
wherein the concentration of methyl methacrylate is from 99 to 99.99% by mass,

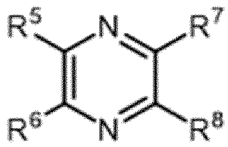

(2)

wherein, in Formula (2) above, $R^5$, $R^6$, $R^7$, and $R^8$ each independently represent a hydrogen atom, an alkyl group, an alkenyl group having a carbon number of 3 or more, an aryl group, a hydroxy group, an alkoxy group, an amino group, a monovalent group containing a carbonyl group, an alkylthio group, or an arylthio group.

**17.** The methyl methacrylate-containing composition according to claim 16, wherein when the concentration of the component A2 is MA2 (umol/L), the MA2 is from 1 to 65,000 pmol/L.

**18.** The methyl methacrylate-containing composition according to claim 17, wherein the MA2 is from 10 to 30,000 pmol/L.

**19.** The methyl methacrylate-containing composition according to claim 16, wherein the molecular weight of the component A2 is 1,000 or less.

**20.** The methyl methacrylate-containing composition according to claim 16, wherein in the Formula (2), $R^5$, $R^6$, $R^7$, and $R^8$ are each independently a hydrogen atom, a $C_{1-5}$ alkyl group, a $C_{3-5}$ alkenyl group, a $C_{1-12}$ aryl group, a $C_{1-6}$ alkoxy group, a $C_{0-6}$ amino group, a $C_{1-6}$ monovalent group containing a carbonyl group, a $C_{1-5}$ alkylthio group, or a $C_{6-10}$ arylthio group.

**21.** The methyl methacrylate-containing composition according to claim 16, wherein in the Formula (2), $R^5$, $R^6$, $R^7$, and $R^8$ are each independently a hydrogen atom, a $C_{1-5}$ alkyl group, or a $C_{1-6}$ alkoxy group.

**22.** The methyl methacrylate-containing composition according to claim 16, wherein in the Formula (2), $R^5$, $R^6$, $R^7$, and $R^8$ are each independently a hydrogen atom, a methyl group, an ethyl group, an n-propyl group, an isopropyl group, or a methoxy group.

**23.** The methyl methacrylate-containing composition according to claim 16, wherein the concentration of methyl methacrylate is from 99.8 to 99.99% by mass.

**24.** The methyl methacrylate-containing composition according to claim 16, wherein the composition does not contain diacetyl, or the concentration of diacetyl contained is 55 pmol/L or less.

**25.** A methyl methacrylate-containing composition, comprising:

methyl methacrylate;
a pyrazine compound represented by the following Formula (1) (component A1); and
an ester compound having an $\alpha$-hydrogen represented by the following Formula (3) (component B2),
wherein the concentration of methyl methacrylate is from 99 to 99.99% by mass,

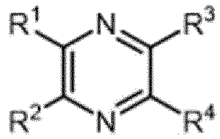

(1)

wherein, in Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group;

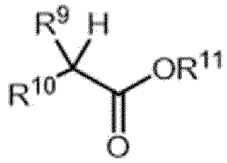

(3)

wherein in Formula (3) above, $R^9$ and $R^{10}$ each independently represent a hydrogen atom, an alkyl group, an alkenyl group, a hydroxy group, an alkoxy group, an amino group, a monovalent group containing a carbonyl group, or an alkylthio group;
$R^{11}$ represents an alkyl group or an aryl group; and
$R^9$ and $R^{10}$, $R^{10}$ and $R^{11}$, $R^{11}$ and $R^9$ each may be linked to each other to form a ring.

**26.** The methyl methacrylate-containing composition according to claim 25, wherein when the concentration of the component A1 is MA1 (umol/L), and the concentration of the component B2 is MB2 (umol/L), the MB2/MA1 is from 0.01 to 1,000.

**27.** The methyl methacrylate-containing composition according to claim 25, wherein when the concentration of the component A1 is MA1 (umol/L), the MA1 is from 1 to 65,000 pmol/L.

**28.** The methyl methacrylate-containing composition according to claim 25, wherein when the concentration of the component B2 is MB2 (umol/L), the MB2 is from 1 to 50,000 pmol/L.

**29.** The methyl methacrylate-containing composition according to claim 25, wherein in the Formula (3), $R^9$ and $R^{10}$ are each independently a hydrogen atom, a $C_{1-5}$ alkyl group, a hydroxy group, a $C_{1-6}$ alkoxy group, a $C_{0-6}$ amino group, a $C_{1-6}$ monovalent group containing a carbonyl group, or a $C_{1-5}$ alkylthio group, and $R^{11}$ is a $C_{1-5}$ alkyl group or a $C_{1-12}$ aryl group.

**30.** The methyl methacrylate-containing composition according to claim 25, wherein the concentration of methyl methacrylate is from 99.8 to 99.99% by mass.

**31.** A methyl methacrylate-containing composition, comprising:

methyl methacrylate;
a pyrazine compound represented by the following Formula (1) (component A1); and
an $\alpha,\beta$-unsaturated carbonyl compound represented by the following Formula (4) (component B3),
wherein the concentration of methyl methacrylate is from 99 to 99.99% by mass,

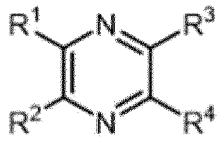

(1)

wherein, in Formula (1) above, $R^1$, $R^2$, $R^3$, and $R^4$ each independently represent a hydrogen atom, an alkyl group, an alkenyl group, an aryl group, a hydroxy group, an alkoxy group, an amino group, a monovalent group containing a carbonyl group, an alkylthio group, or an arylthio group;

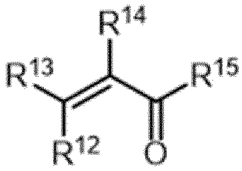

(4)

wherein, in Formula (4) above, $R^{12}$, $R^{13}$, and $R^{14}$ each independently represent a hydrogen atom, an alkyl group, an alkenyl group, an aryl group, a hydroxy group, an alkoxy group, an amino group, a monovalent group containing a carbonyl group, an alkylthio group, or an arylthio group;
$R^{15}$ represents an alkyl group, an alkenyl group, an aryl group, a hydroxy group, an alkoxy group, an amino group, a monovalent group containing a carbonyl group, an alkylthio group, or an arylthio group;
$R^{12}$ and $R^{13}$, $R^{13}$ and $R^{14}$, and $R^{14}$ and $R^{15}$ each may be linked to each other to form a ring,
except when $R^{12}$ = H, $R^{13}$ = H, $R^{14}$ = $CH_3$, and $R^{15}$ = $OCH_3$, that is, when the $\alpha,\beta$-unsaturated carbonyl compound represented by Formula (4) is methyl methacrylate; and
H represents a hydrogen atom, C represents a carbon atom, and O represents an oxygen atom.

**32.** The methyl methacrylate-containing composition according to claim 31, wherein when the concentration of the component A1 is MA1 (umol/L), and the concentration of the component B3 is MB3 (umol/L), the MB3/MA1 is from 0.01 to 1,000.

**33.** The methyl methacrylate-containing composition according to claim 31, wherein when the concentration of the component A1 is MA1 (umol/L), the MA1 is from 1 to 65,000 pmol/L.

**34.** The methyl methacrylate-containing composition according to claim 31, wherein when the concentration of the component B3 is MB3 (umol/L), the MB3 is from 1 to 85,000 pmol/L.

**35.** The methyl methacrylate-containing composition according to claim 31, wherein in Formula (4) above, $R^{12}$, $R^{13}$, and $R^{14}$ are each independently a hydrogen atom, a $C_{1-5}$ alkyl group, a $C_{2-5}$ alkenyl group, a $C_{1-12}$ aryl group, a $C_{1-6}$ alkoxy group, a $C_{0-6}$ amino group, a $C_{1-6}$ monovalent group containing a carbonyl group, or a $C_{1-5}$ alkylthio group; and $R^{15}$ is a $C_{1-5}$ alkyl group, a $C_{2-5}$ alkenyl group, a $C_{1-12}$ aryl group, a hydroxy group, a $C_{1-6}$ alkoxy group, a $C_{0-6}$ amino group, a $C_{1-6}$ monovalent group containing a carbonyl group, or a $C_{1-5}$ alkylthio group.

**36.** The methyl methacrylate-containing composition according to claim 31, wherein the concentration of methyl methacrylate is from 99.8 to 99.99% by mass.

**37.** A method of storing a methyl methacrylate-containing composition, in which the methyl methacrylate-containing composition according to any one of claims 1 to 36 is stored at 0 to 50°C.

**38.** A method of producing a methyl methacrylate polymer, comprising a step of polymerizing a polymeric composition comprising a methyl methacrylate-containing composition according to any one of claims 1 to 36.

**39.** The method of producing a methyl methacrylate polymer according to claim 38, wherein the polymeric composition comprises a monomer that can be copolymerized with methyl methacrylate.

## INTERNATIONAL SEARCH REPORT

International application No.
**PCT/JP2022/043975**

**A. CLASSIFICATION OF SUBJECT MATTER**

***C07C 69/54***(2006.01)i; ***C07C 39/06***(2006.01)i; ***C07D 241/12***(2006.01)i; ***C08F 2/44***(2006.01)i; ***C08F 20/14***(2006.01)i
FI: C07C69/54 Z; C07C39/06; C07D241/12; C08F20/14; C08F2/44 B

According to International Patent Classification (IPC) or to both national classification and IPC

**B. FIELDS SEARCHED**

Minimum documentation searched (classification system followed by classification symbols)

C07C69/54; C07C39/06; C07D241/12; C08F2/44; C08F20/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 48-81806 A (TOAGOSEI CHEMICAL INDUSTRY CO., LTD.) 01 November 1973 (1973-11-01)<br>p. 3, lower left column, line 3 | 1-39 |
| A | JP 8-169862 A (ELF ATOCHEM SA) 02 July 1996 (1996-07-02)<br>paragraphs [0009], [0012] | 1-39 |
| A | JP 2004-155757 A (ROHM & HAAS CO) 03 June 2004 (2004-06-03)<br>entire text | 1-39 |
| A | JP 2005-502695 A (BASF AKTIENGESELLSCHAFT) 27 January 2005 (2005-01-27)<br>entire text | 1-39 |
| A | JP 10-504553 A (SHELL INTERNATIONALE RESEARCH MAATSCHAPPIJ B.V.) 06 May 1998 (1998-05-06)<br>entire text | 1-39 |
| A | JP 2002-533309 A (RHODIA CHIMIE) 08 October 2002 (2002-10-08)<br>entire text | 1-39 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 January 2023** | **31 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**

International application No.
**PCT/JP2022/043975**

**C. DOCUMENTS CONSIDERED TO BE RELEVANT**

| Category* | Citation of document, with indication, where appropriate, of the relevant passages | Relevant to claim No. |
|---|---|---|
| A | JP 2002-513034 A (RHODIA CHIMIE) 08 May 2002 (2002-05-08) entire text | 1-39 |

Form PCT/ISA/210 (second sheet) (January 2015)

**INTERNATIONAL SEARCH REPORT**
**Information on patent family members**

International application No.
**PCT/JP2022/043975**

| Patent document cited in search report | Publication date (day/month/year) | Patent family member(s) | Publication date (day/month/year) |
|---|---|---|---|
| JP 48-81806 A | 01 November 1973 | (Family: none) | |
| JP 8-169862 A | 02 July 1996 | US 5585514 A<br>examples 2, 8<br>EP 690041 A1<br>KR 10-1996-0000851 A<br>CN 1125713 A | |
| JP 2004-155757 A | 03 June 2004 | US 2003/0208093 A1<br>entire text<br>EP 1359137 A1<br>CN 1454886 A<br>KR 10-0967581 B1 | |
| JP 2005-502695 A | 27 January 2005 | US 2004/0236143 A1<br>entire text<br>WO 2003/022793 A1<br>EP 1427692 A1<br>CN 1553889 A | |
| JP 10-504553 A | 06 May 1998 | US 5968322 A<br>entire text<br>WO 1996/006066 A1<br>EP 804407 A1<br>KR 10-1997-0704662 A | |
| JP 2002-533309 A | 08 October 2002 | WO 2000/037412 A1<br>EP 1140772 A1<br>CN 1331669 A<br>KR 10-2001-0103717 A | |
| JP 2002-513034 A | 08 May 2002 | WO 1999/055797 A1<br>EP 1080163 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004155757 A
- JP 2005502695 A
- JP H10504553 A
- JP 2002533309 A
- JP 2002513034 A
- JP S5037882 A


### Non-patent literature cited in the description


- **TORU KURODA.** Development of Catalyst for Producing Methyl Methacrylate. *Catalysts & Catalysis,* 2003, vol. 45 (5), 366-371
- **TAKAYUKI OTSU.** On the Functions of Polymerization Inhibitors. *Synthetic Organic Chemistry,* 1975, vol. 33 (8), 634-640